# EUROPEAN PATENT APPLICATION

(11) **EP 4 809 903 A1**
(43) Date of publication of application: **23.09.2026**
(21) Application number: 24891060.6
(22) Date of filing: 02.09.2024
(51) Int. Cl.: A61B 1/008

(54) **BENDING MECHANISM AND ENDOSCOPE**

(30) Priority: 15.11.2023 US 202363599292 P
(71) Applicant: Olympus Medical Systems Corp., Tokyo 192-8507 (JP)
(72) Inventor: HIRASAWA Yoshihiro, Hachioji-shi, Tokyo 192-8507 (JP); YAGI Hitoshi, Hachioji-shi, Tokyo 192-8507 (JP)
(74) Representative: Schicker, Silvia
(86) International application number: PCT/JP2024/031491
(87) International publication number: WO 2025/105017

(57) **Abstract**

A bending mechanism (20) includes a first tube (2b), a first guide (24), and a second guide (22). The first tube (2b) is formed along an axis (O), and is bent by a wire (23) being pulled. The first guide (24) is formed in a cylindrical shape with a resin, and provided in the first tube (2b) along the axis (O), the first guide being configured to hold the wire (23). The second guide (22) is fixed to the first tube (2b), and configured to hold the first guide (24).

## Description

### Technical Field

The present disclosure relates to a bending mechanism configured to be bent by a wire being pulled, and an endoscope including the bending mechanism.

### Background Art

Conventionally, endoscopes that include a bending mechanism capable of changing a direction of a distal end portion have been proposed. The bending mechanism is configured by arranging a plurality of bending pieces swingably connected to each other in a bending portion, and fixing a distal end of a bending wire to a bending piece arranged at a distal-most position, for example. The bending wire is inserted through wire receivers that are provided respectively to the bending pieces in the bending portion. The bending wire is inserted through a tubular portion on a proximal end side of the bending portion, and a proximal end side of the bending wire is connected to a drum and the like in an operation portion. The drum in the operation portion rotates in conjunction with a bending operation knob, to pull the bending wire. As a result, operating the bending operation knob causes the bending portion to bend.

International Publication No. WO2016-167099, for example, recites a bending tube for endoscope configured by connecting a plurality of ring members by using a plurality of tubular members. Specifically, the technology recited in the publication employs a configuration of connecting a plurality of bending pieces by using the tubular members, instead of a common configuration of connecting a plurality of bending pieces by using hinges, or the like.

When an insertion portion of an endoscope is inserted into a subject, there is a case where a bending portion becomes a complicated shape such as a loop shape. If a bending wire is pulled in such a state, friction and catching occur between the bending wire and the wire receivers, which results in an increase in a sliding resistance.

Then, a loss occurs in a pulling force of the bending wire at the wire receivers, and an amount of operation force required for operating the bending operation knob increases according to the sliding resistance, which results in a deterioration in the operability. In addition, abrasion, scraping, etc., sometimes occur at a corner portion of a wire receiver with which the bending wire is in contact.

To address such circumstances, a technology is known in which a powder lubricant is used to reduce sliding resistance, thereby enabling bending operation to be performed easily with a small amount of operation force.

In general, endoscopes are manufactured in a controlled area such as a clean room, but a powder lubricant cannot be brought into such a controlled area. For this reason, using the powder lubricant reduces a degree of freedom of manufacturing and increases manufacturing costs, which results in complication of a construction of an assembly line.

The present disclosure has been achieved in view of the above-described circumstances, and an object of the present disclosure is to provide an inexpensive bending mechanism that enables bending operation to be performed easily with a small amount of operation force, and an endoscope including the bending mechanism.

### Disclosure of Invention

### Means for Solving the Problem

A bending mechanism according to one aspect of the present disclosure includes: a first tube formed along an axis extending from a first side to a second side, the first tube being configured to be bent by a wire being pulled; a first guide made of a resin and formed in a cylindrical shape, the first guide being provided in the first tube along the axis and configured to hold the wire; and a second guide fixed to the first tube, and configured to hold the first guide.

An endoscope according to one aspect of the present disclosure includes: an insertion portion configured to be inserted into a subject; an operation portion provided on a first side relative to the insertion portion; and a bending mechanism, at least a part of which is provided on a second side of the insertion portion. The bending mechanism includes: a first tube formed along an axis extending from the first side to the second side, the first tube being configured to be bent by a wire being pulled; a first guide made of a resin and formed in a cylindrical shape, the first guide being provided in the first tube along the axis and configured to hold the wire; and a second guide fixed to the first tube, and configured to hold the first guide, and the first side of the wire is connected to the operation portion.

### BRIEF DESCRIPTION OF THE DRAWINGS

FIG. 1 shows a configuration example of an endoscope according to respective embodiments of the present disclosure.
FIG. 2 is a perspective view showing an example of a flexible tube in a first example of a related technology.
FIG. 3 is a perspective view showing a modified example of the flexible tube in the first example of the related technology.
FIG. 4 shows a configuration, in a planarly developed state, of an outer circumferential surface of the flexible tube in the first example of the related technology.
FIG. 5 shows a configuration, in a planarly developed state, of an outer circumferential surface of a flexible tube in a second example of the related technology.
FIG. 6 is a perspective view showing a flexible tube in a third example of the related technology.
FIG. 7 shows a configuration, in a planarly developed state, of an outer circumferential surface of the flexible tube in the third example of the related technology.
FIG. 8 shows a configuration, in a planarly developed state, of an outer circumferential surface of a flexible tube in a fourth example of the related technology.
FIG. 9 shows a configuration, in a planarly developed state, of an outer circumferential surface of a flexible tube in a fifth example of the related technology.
FIG. 10 shows a configuration, in a planarly developed state, of an outer circumferential surface of a flexible tube in a sixth example of the related technology.
FIG. 11 shows a configuration, in a planarly developed state, of an outer circumferential surface of a flexible tube in a seventh example of the related technology.
FIG. 12 shows a configuration, in a planarly developed state, of an outer circumferential surface of a flexible tube in an eighth example of the related technology.
FIG. 13 shows a configuration, in a planarly developed state, of an outer circumferential surface of a flexible tube in a ninth example of the related technology.
FIG. 14 shows a configuration, in a planarly developed state, of an outer circumferential surface of a flexible tube in a tenth example of the related technology.
FIG. 15 is a perspective view showing a flexible tube in an eleventh example of the related technology.
FIG. 16 is a perspective view showing a flexible tube in a twelfth example of the related technology.
FIG. 17 is a perspective view showing a flexible tube in a thirteenth example of the related technology.
FIG. 18 is a cross-sectional view showing a configuration example of a bending wire in a bending portion in a first embodiment of the present disclosure.
FIG. 19 is a partial perspective view showing the configuration example of the bending wire in the bending portion in the first embodiment.
FIG. 20 is a chart showing a comparison of a relationship between the bending wire inserted into a tube and a wire receiver, in the first embodiment, and a relationship between a conventional bending wire and a wire receiver.
FIG. 21 is a cross-sectional view showing a configuration of tubes through which bending wires are respectively inserted, in the bending portion in a straight state, in the first embodiment.
FIG. 22 is a cross-sectional view showing a state of the tubes through which the bending wires are respectively inserted, in the bending portion in a maximum bending state, in the first embodiment.
FIG. 23 is a cross-sectional view showing a configuration of tubes through which bending wires are respectively inserted, in a bending portion in a straight state, in a second embodiment of the present disclosure.
FIG. 24 is a cross-sectional view showing a state of the tubes through which the bending wires are respectively inserted, in the bending portion in a maximum bending state, in the second embodiment.
FIG. 25 is a cross-sectional view parallel to a center axis, which shows a modified example of a partial tube constituting an intermittent tube, in the second embodiment.
FIG. 26 is a cross-sectional view vertical to the center axis, which shows a configuration of tubes through which bending wires are respectively inserted, in a third embodiment of the present disclosure.
FIG. 27 is a chart showing a configuration example in which wire receivers are provided integrally with a bending piece, in a fourth embodiment of the present disclosure.
FIG. 28 shows a configuration of tubes through which bending wires are respectively inserted, in a fifth embodiment of the present disclosure.
FIG. 29 shows a configuration of tubes through which bending wires are respectively inserted, in a sixth embodiment of the present disclosure.
FIG. 30 shows a configuration of tubes through which bending wires are respectively inserted, in a seventh embodiment of the present disclosure.
FIG. 31 shows a state of a tube, when a bending portion is bent, in an eighth embodiment of the present disclosure.
FIG. 32 is a perspective view showing a first configuration example of the tube in the eighth embodiment.
FIG. 33 is a chart showing a cross section and a partially enlarged lateral surface of the tube in the first configuration example in the eighth embodiment.
FIG. 34 is a perspective view showing a second configuration example of the tube in the eighth embodiment.
FIG. 35 is a chart showing a cross section and a partially enlarged lateral surface of the tube in the second configuration example, in the eighth embodiment.
FIG. 36 is a partial perspective view showing a configuration of a wire receiver in a bending piece, in a ninth embodiment of the present disclosure.
FIG. 37 is a partial perspective view showing a configuration of a wire receiver in a bending piece in a modified example of the ninth embodiment.

### Best Mode for Carrying Out the Invention

Hereinafter, embodiments of the present disclosure will be described with reference to drawings. However, the present disclosure is not limited by the embodiments to be described below.

Note that in the illustrations in the drawings, the same or corresponding elements are attached with the same reference signs as appropriate. In addition, the drawings are schematic, and care should be taken to the fact that a relationship among the lengths of the respective elements, a ratio of the lengths of the respective elements, the number of the respective elements, and the like are sometimes different from the actual ones for simplification of the description. Furthermore, the respective drawings sometimes include parts in which the relationships and ratios among the lengths of the elements, the number of the elements, and the like are different.

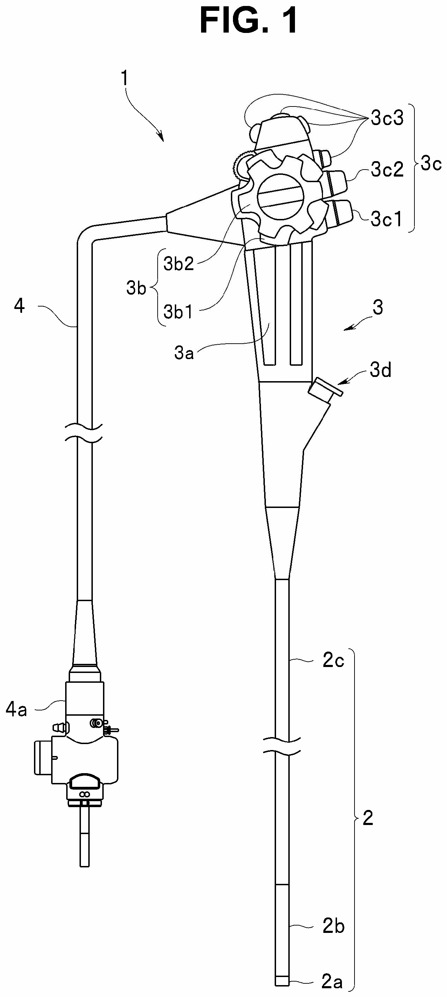

FIG. 1 shows a configuration example of an endoscope 1 according to respective embodiments of the present disclosure.

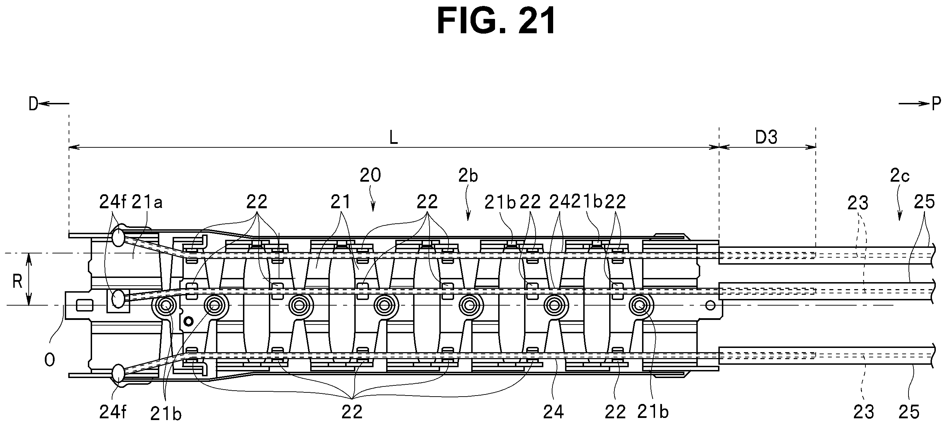

The endoscope 1 is a device configured to perform observation and treatment of a subject. The endoscope 1 includes an insertion portion 2 configured to be inserted into a subject, an operation portion 3 provided on a proximal end side (a first side: a proximal end direction P side in FIG. 21) of the insertion portion 2, and a universal cord 4 extended from the operation portion 3.

Note that the subject into which the insertion portion 2 is inserted is supposed to be a living thing such as a human being or an animal, but not limited thereto. The subject may be a non-living thing such as a machine or architecture. In addition, the endoscope 1 may be an automatically inserting endoscope configured to advance or retreat by rotation. The endoscope 1 may be any of an upper gastrointestinal endoscope, a lower gastrointestinal endoscope, or an endoscope to be applied to other parts.

The insertion portion 2 includes, in the following order from the distal end toward the proximal end, a distal end portion 2a, a bending portion 2b (a bending tube, a first tube), and a tubular portion 2c (a second tube).

The distal end portion 2a includes an observation system and an illumination system, for example. The illumination system includes an illumination optical system and the like, and applies illumination light to a subject. The observation system includes, inside an observation window, an objective optical system and an image pickup device. The observation system forms an image of return light from the subject by the objective optical system and picks up the image by the image pickup device.

The bending portion 2b is provided on the proximal end side of the distal end portion 2a, and configured to be bendable in two directions, i.e., up and down directions, or in four directions, i.e., up, down, left, and right directions, for example. When the bending portion 2b is bent, a direction of the distal end portion 2a is changed, and a direction of observation by the observation system and an irradiation direction of illumination light by the illumination system are changed. In addition, the bending portion 2b is bent also for improving insertion performance of the insertion portion 2 in the subject.

The tubular portion 2c is a tubular part connecting the proximal end of the bending portion 2b and the distal end of the operation portion 3. The tubular portion 2c has a flexible form that sags according to the shape of the subject into which the tubular portion 2c is inserted. In this case, the endoscope 1 is called a flexible endoscope.

The operation portion 3 is provided on the proximal end side of the insertion portion 2, and is a part with which various operations related to the endoscope 1 are performed by being grasped by the hand. The operation portion 3 includes, for example, a grasping portion 3a, a bending operation knob 3b, a plurality of operation buttons 3c, and a treatment instrument insertion port 3d.

The grasping portion 3a is a part for an operator to grasp the endoscope 1 with the palm.

The bending operation knob 3b is an operation device for performing bending operation of the bending portion 2b by using the thumb of the hand grasping the grasping portion 3a, for example. When the bending portion 2b is bendable in four directions, i.e., up, down, left, and right directions, the bending operation knob 3b includes a UD bending operation knob 3b1 for bending operation in the up and down directions, and an RL bending operation knob 3b2 for bending operation in the left and right directions.

The plurality of operation buttons 3c include, for example, a gas/liquid feeding button 3c1, a suction button 3c2, and other buttons 3c3, for example.

The gas/liquid feeding button 3c1 is a button for performing gas/liquid feeding to the observation window provided on the distal end surface of the observation system via a gas/liquid feeding channel, not shown, to thereby perform operation for cleaning the observation window.

The suction button 3c2 is a button for performing an operation for sucking a liquid, a mucosa, and the like from the subject via a suction channel, not shown.

The other buttons 3c3 are used as a freeze button for temporarily stopping a monitor screen, a release button for picking up a still image, a switching button for switching to special light, and the like, for example.

The treatment instrument insertion port 3d is provided on a distal-end-side lateral surface of the grasping portion 3a. The treatment instrument insertion port 3d is communicated with a treatment instrument channel. The treatment instrument channel includes a distal-end-side opening at the distal end portion 2a. When various treatment instruments such as forceps are inserted from the treatment instrument insertion port 3d, the distal end of the treatment instrument protrudes from the distal-end-side opening of the treatment instrument channel, to enable various kinds of treatment to be performed on the subject.

The universal cord 4 is extended from the proximal-end-side lateral surface of the operation portion 3, for example. The universal cord 4 includes, at the extension end thereof, a connector 4a. The connector 4a is connected to an endoscope processor and a light source apparatus (or an endoscope processor which functions also as the light source apparatus), which are not shown.

The endoscope processor is configured to transmit a driving signal and power to the image pickup device in the distal end portion 2a. In addition, the endoscope processor is configured to receive an image pickup signal obtained by picking up an image of the subject by the image pickup device. The light source apparatus emits illumination light to transmit the illumination light via a light guide not shown. The illumination light transmitted by the light guide is applied from the distal end surface of the distal end portion 2a to the subject.

Note that, instead of the configuration in which the illumination light emitted by the light source apparatus is transmitted by the light guide, a configuration may be employed in which a light-emitting element is provided in the distal end portion 2a, power is supplied from the endoscope processor to the light-emitting element, and the light-emitting element emits the illumination light.

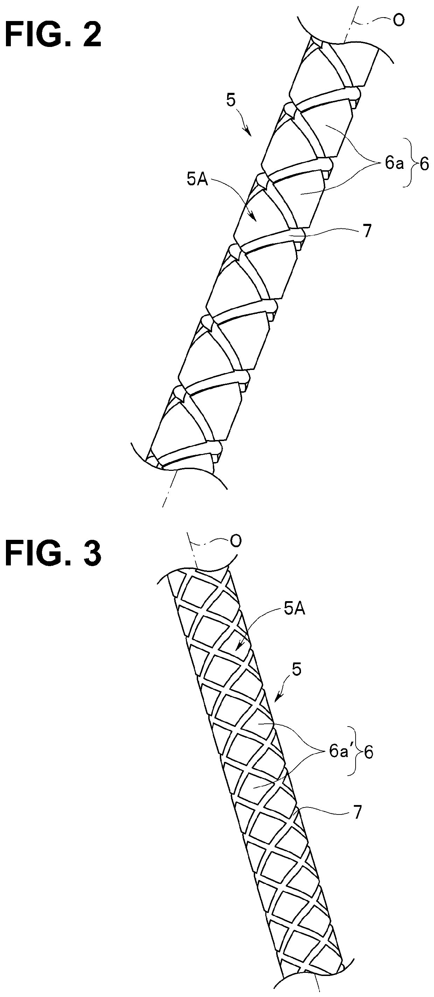

Next, with reference to FIGS. 2 to 17, description will be made on a flexible tube 5 that is used in the endoscope 1, as a related technology.

As examples of flexible tubes, corrugated tubes have been known conventionally. Corrugated tubes include a bellows structure in which ring-shaped protruded portions around an axis and ring-shaped recessed portions around the axis are alternately formed along a center axis in a longitudinal direction. Corrugated tubes can be manufactured inexpensively, have a characteristic of high bendability, and are used for a covering material of an electric wire, for example.

Incidentally, flexible endoscopes include an insertion portion configured as a tube having flexibility. In recent years, single-use endoscopes, which are disposed of after a single use, have been proposed. Such single-use endoscopes can preferably be manufactured inexpensively in view of costs, and a use of corrugated tubes are considered.

In general, characteristics required for an insertion portion of an endoscope include bendability, an extension/contraction resistance, and a torque resistance. The bendability is, as is well known, bending performance. The extension/contraction resistance is a resistance to extension and contraction. Specifically, the extension/contraction resistance includes a compression resistance which is a resistance to compression and a tension resistance which is a resistance to tension. In addition, the torque resistance is a resistance to twisting around a center axis.

A corrugated tube having a bellows structure has a bendability and a high torque resistance. However, the corrugated tube has a low extension/contraction resistance, and contracts upon receipt of a compression force and extends upon receipt of a pulling force.

The above-described several characteristics are in a trade-off relationship with one another in many cases. For this reason, it is difficult to increase the extension/contraction resistance and the torque resistance of the corrugated tube having the bellows structure, while maintaining the bendability thereof. In addition, in the corrugated tube having the bellows structure, it is also difficult to more freely control the characteristics such as viscoelasticity at the time of bending.

In the related technology to be described below, description will be made on an endoscope including a flexible tube that can be manufactured inexpensively, while balancing a bendability, an extension/contraction resistance, and a torque resistance thereof.

### [First Example of Related Technology]

FIG. 2 to FIG. 4 show a first example of the related technology. FIG. 2 is a perspective view showing an example of a flexible tube 5 in the first example of the related technology.

The endoscope 1 includes the flexible tube 5. The flexible tube 5 is provided, for example, in the insertion portion 2 (that is, the part including the bending portion 2b and the tubular portion 2c) of the endoscope 1. However, the configuration is not limited to the above, and the flexible tube 5 may be provided in the universal cord 4. Furthermore, the flexible tube 5 is not limited to be used for the endoscope 1, and can be widely applied to medical devices. For example, the flexible tube 5 may be applied to a treatment instrument such as a catheter, an overtube, and the like.

The flexible tube 5 is a tube having a flexibility, which is along a center axis O extending from one end to the other end. The flexible tube 5 includes, on an outer circumferential surface 5A thereof, a plurality of protruded surfaces 6 and recessed surfaces 7 defined by the plurality of protruded surfaces 6. The recessed surfaces 7 are each a bottomed surface and different from a slit.

The flexible tube 5 is formed by feeding a heated material such as plastic from an extruder into a cylindrical-shaped mold, and by making the material closely contact the inner circumferential surface of the mold by a vacuum mechanism provided in the mold. The cylindrical-shaped mold is constituted of a pair of molds which are divided into two by a plane passing through the center axis, for example. The pair of molds is used by being transported like an endless crawler, for example.

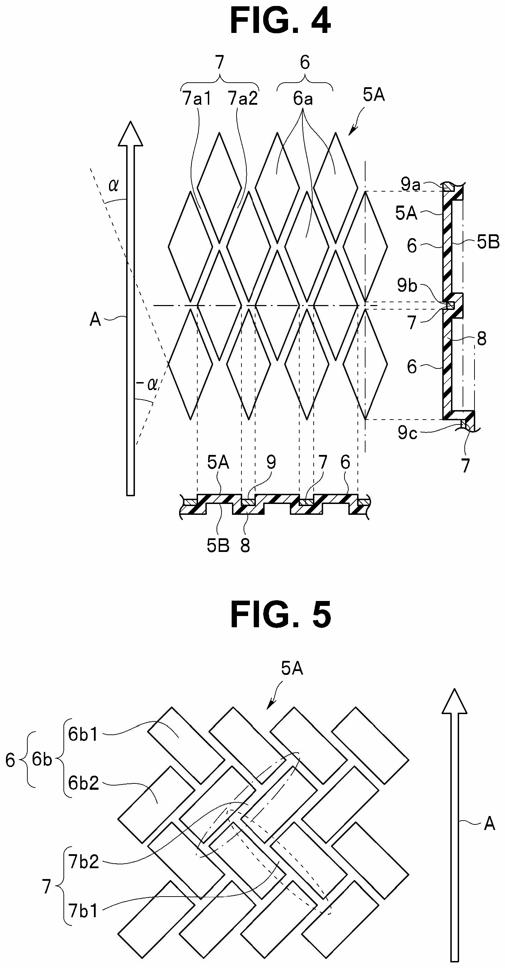

Therefore, the shapes of the protruded surfaces 6 and the recessed surfaces 7 that are formed on the outer circumferential surface 5A of the flexible tube 5 are defined accurately in accordance with the mold. On the other hand, an uneven shape of the inner circumferential surface 5B of the flexible tube 5 (see FIG. 4) is roughly formed according to the material that is made to closely contact the inner circumferential surface with a substantially even thickness. In other words, when viewing from the inner circumferential surface 5B of the flexible tube 5, the parts corresponding to the protruded surfaces 6 are recessed surfaces and the part corresponding to the recessed surfaces 7 are protruded surfaces.

The plurality of protruded surfaces 6 are each surrounded by the recessed surfaces 7, to be isolated from one another. In other words, the plurality of protruded surfaces 6 are formed intermittently in the axial direction parallel to the center axis O and in the circumferential direction around the center axis O.

The plurality of protruded surfaces 6 each have the same shape, for example, (note that description will be made later on the example in which the plurality of protruded surfaces have different shapes) and arranged periodically along the outer circumferential surface 5A of the flexible tube 5. Each of the plurality of protruded surfaces 6 forms a polygon, in particular, a quadrangle in the present example, when the outer circumferential surface 5A of the flexible tube 5 is planarly developed.

Specifically, in the example shown in FIG. 2, each of the plurality of protruded surfaces 6 is a rhombus protruded surface 6a that forms a rhombus when the outer circumferential surface 5A of the flexible tube 5 is planarly developed. Note that the "rhombus protruded surface" is hereinafter just referred to as "rhombus", and the recitation of "protruded surface" will be omitted. Similarly, also protruded surfaces having other shapes, which will be described later, are recited by omitting the recitation of "protruded surfaces".

As is known, the rhombus 6a has four sides having the same length. Except for the case where the rhombus 6a is a square, one of two diagonal lines is longer than the other.

The protruded surfaces 6 and the recessed surfaces 7 are formed to be plane symmetrical with the plane passing through the center axis O, for example. In such a configuration, when molding is performed using the mold divided into two, the separation from the mold is easy.

FIG. 3 is a perspective view showing a modified example of the flexible tube 5 in the first example of the related technology.

When the diameter of the flexible tube 5 shown in FIG. 2 and the diameter of the flexible tube 5 shown in FIG.3 are the same, for example, an area of a rhombus 6a' shown in FIG. 3 is relatively smaller than an area of the rhombus 6a shown in FIG. 2. For the rhombuses 6a shown in FIG. 2, two rhombuses are arranged in the circumferential direction, while for the rhombuses 6a' shown in FIG. 3, four rhombuses are arranged in the circumferential direction, for example. The number of the protruded surfaces 6 arranged in the circumferential direction is not limited to an even number and may be an odd number, or may be an appropriate number.

FIG. 4 shows a configuration, in a planarly developed state, of the outer circumferential surface 5A of the flexible tube 5 in the first example of the related technology. Note that FIG. 4 shows also a cross section in the axial direction and a cross section in the circumferential direction of the flexible tube 5 that is planarly developed.

The rhombuses 6a in the example shown in FIG. 2 are subjected to periodic tiling (however, tiling except for the recessed surfaces 7, the same applies hereafter) such that, for example, the longer diagonal line is parallel to the axial direction (direction of the arrow A shown in FIG. 4).

Then, the recessed surfaces 7 are all inclined with respect to the axial direction, and inclined with respect to the circumferential direction (direction vertical to the arrow A in the developed view in FIG. 4).

Specifically, the recessed surfaces 7 include a first inclined part 7a1 inclined at an angle α with respect to the axial direction and a second inclined part 7a2 inclined at an angle -α with respect to the axial direction. Here, α is an angle of 45 degrees or less. The first inclined part 7a1 and the second inclined part 7a2 are spiral-shaped recessed surfaces 7 in the flexible tube 5.

Note that α is not limited to 45 degrees or less, and it may be set to an angle larger than 45 degrees as required by a design.

For example, if α is set to 45 degrees or less, compared to the case where α is set to larger than 45 degrees, the torque resistance becomes slightly low, but the extension/contraction resistance can be increased. Therefore, α may be set to 45 degrees or less in the case where an emphasis is placed on suppressing the extension or the contraction of the flexible tube 5 when the flexible tube 5 has received a compression force or a pulling force.

Conversely, when α is set to larger than 45 degrees, compared to the case where α is set to 45 degrees or less, the extension/contraction resistance becomes slightly low, but the torque resistance can be increased. Therefore, α may be set to larger than 45 degrees in the case where an emphasis is placed on suppressing the twisting of the flexible tube 5 when the flexible tube 5 has received a twisting force around the center axis O.

Thus, controlling the angle α enables control of determining a balance between the extension/contraction resistance and the torque resistance that are in the trade-off relationship with each other.

In addition, as shown in the cross section of FIG. 4, the recessed surfaces 7 may be filled with a filling material to form filling structure parts 9.

In other words, the flexible tube 5 may include a tube main body 8 and the filling structure parts 9.

The tube main body 8 is formed by using a material having a first Young's modulus so as to include the plurality of protruded surfaces 6 and the recessed surfaces 7 by the above-described mold.

The filling structure parts 9 are formed by filling the recessed surfaces 7 of the tube main body 8 with the filling material having a second Young's modulus lower than the first Young's modulus.

Further, a filling rate of the filling material in the filling structure parts 9 may be made different along the axial direction. In the example shown in the cross section in the axial direction in FIG. 4, an example is shown in which the filling rate is decreased sequentially in the axial direction in the order of a proximal-end-side filling structure part 9a, a middle filling structure part 9b, and a distal-end-side filling structure part 9c.

Note that as shown in the cross section in the circumferential direction in FIG. 4, the filling rate of the filling material in the circumferential direction is constant. However, in order to make the easiness of bending different according to the bending direction, the filling rate of the filling material in the circumferential direction may be changed.

In addition, as shown in FIG.4, a distance from the recessed surface 7 to the protruded surface 6 in the radial direction, with the center axis O as a center (the depth of the recessed surface 7 with respect to the protruded surface 6 or the height of the protruded surface 6 with respect to the recessed surface 7) may be made different according to the position of each of the recessed surfaces 7 on the flexible tube 5. In the example shown in FIG. 4, the depth of the recessed surface 7 at a position where the filling structure part 9c is provided is set to be deeper than the depths of the respective recessed surfaces 7 at positions where the filling structure parts 9a and 9b are respectively provided. Note that the depth from the protruded surface 6 to the recessed surface 7 may be made different according to the angle of the recessed surface 7 with respect to the axial direction.

Thus, the filling structure parts 9 are provided and the depths of the recessed surfaces 7 are adjusted according to the positions thereof on the flexible tube 5, which enables the characteristics of the flexible tube 5, such as the bendability and the viscoelasticity at the time of bending to be controlled more freely.

According to the first example of the related technology, the flexible tube 5 has a structure including the plurality of protruded surfaces 6 each surrounded by the recessed surfaces 7 such that the plurality of protruded surfaces 6 are isolated from one another. Such a configuration enables the flexible tube 5 in the present example to achieve the characteristics which cannot be achieved by the corrugated tube having the bellows structure in which ring-shaped protruded portions and ring-shaped recessed portions are alternately formed along the direction of the center axis O.

The flexible tube 5 in the present example has a surface smoothness which is a little higher than that of the corrugated tube having the bellows structure. In particular, when the filling structure parts 9 are formed respectively in the recessed surfaces 7, the surface smoothness can be more enhanced, which can provide a configuration more suitable for medical devices including the endoscope 1 configured to be inserted into a subject.

The flexible tube 5 in the present example includes the recessed surfaces 7 which are inclined with respect to the axial direction and inclined with respect to the circumferential direction. Such a flexible tube 5 can increase the extension/contraction resistance while maintaining the bendability.

In the flexible tube 5 in the present example, the balance among the bendability, the extension/contraction resistance, and the torque resistance can be controlled more freely. Specifically, various parameters such as the angle α of the recessed surfaces 7, the depth of the recessed surfaces 7 from the protruded surfaces 6, etc., can be adjusted, thereby providing a high degree of freedom in controlling the characteristics.

The filling structure parts 9 are provided respectively in the recessed surfaces 7 by filling the recessed surfaces 7 with the filling material having the Young's modulus lower than that of the tube main body 8, and the filling rates of the filling structure parts 9 are adjusted according to the positions thereof, to thereby be capable of providing an elastic deformation characteristic different from that of the tube main body 8 to the flexible tube 5. Such a configuration can address the case where the tubular portion 2c and the bending portion 2b are intended to have different rigidities, for example. The configuration has a larger number of parameters for controlling the characteristics, which enhances the degree of freedom when the characteristics of the flexible tube 5 are optimized.

The flexible tube 5 in the present example can be manufactured inexpensively by using the extruder, the mold having the vacuum mechanism, etc., similarly to the conventional corrugated tube.

### [Second Example of Related Technology]

FIG. 5 shows a configuration, in a planarly developed state, of an outer circumferential surface 5A of a flexible tube 5 in a second example of the related technology. In the second example of the related technology, the same components as those in the first example of the related technology are attached with the same reference signs and descriptions thereof will be omitted as appropriate. In the second example of the related technology, description will be made mainly on points different from the first example of the related technology.

As shown in FIG. 5, a plurality of protruded surfaces 6 each have the same shape, and form a rectangle 6b when the outer circumferential surface 5A of the flexible tube 5 is planarly developed. As is well known, the rectangle 6b has four corners, all of which have right angles, and except for the case where the rectangle 6b is a square, the rectangle 6b has a pair of opposing long sides and a pair of opposing short sides.

The rectangles 6b constituting the plurality of protruded surfaces 6 each include two kinds of rectangles, i.e., a rectangle 6b1 with long sides thereof being arranged in a direction from the upper left side to the lower right side and a rectangle 6b2 with long sides thereof being arranged in a direction from the upper right side to the lower left side.

In addition, the recessed surfaces 7 include a first inclined part 7b1 (part surrounded by a dotted line in FIG. 5) inclined with respect to the axial direction, along the long side of the rectangle 6b1, and a second inclined part 7b2 (the part surrounded by the one-dot chain line in FIG. 5) inclined with respect to the axial direction, along the long side of the rectangle 6b2.

The first inclined part 7b1 has both ends that abut against the rectangle 6b2. The second inclined part 7b2 has both ends that abut against the rectangle 6b1. Thus, the first inclined parts 7b1 and the second inclined parts 7b2 are formed intermittently, and the length of these in the axial direction is shorter than the length of the flexible tube 5 in the axial direction. The first inclined parts 7b1 and the second inclined parts 7b2 that are formed intermittently have an angle smaller than 360 degrees in the circumferential direction.

The second example of the related technology provides substantially the same effects as those of the first example of the related technology.

In addition, in the first example of the related technology, the spiral-shaped first inclined part 7a1 and the spiral-shaped second inclined part 7a2 are each continuous in the axial direction of the flexible tube 5. In contrast, the first inclined parts 7b1 and the second inclined parts 7b2 in the second example of the related technology are formed intermittently in the axial direction. With such a configuration, in the flexible tube 5 in the second example of the related technology, a degree of freedom in controlling the characteristics such as the bendability, the extension/contraction resistance, and the torque resistance can be more enhanced.

Note that, in FIG. 5, the rectangles 6b are each arranged with the respective sides thereof inclined with respect to the axial direction, but are not limited to such an arrangement. The rectangles 6b may each be arranged with the one pair of opposing sides being along the axial direction and the other pair of opposing sides being along the circumferential direction. In addition, the plurality of respective rectangles 6b are not limited to have the same size. The rectangles 6b may have different sizes according to the positions thereof, and the like.

### [Third Example of Related Technology]

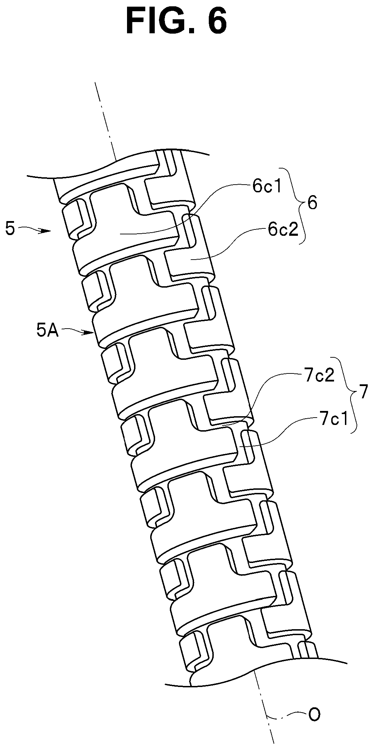

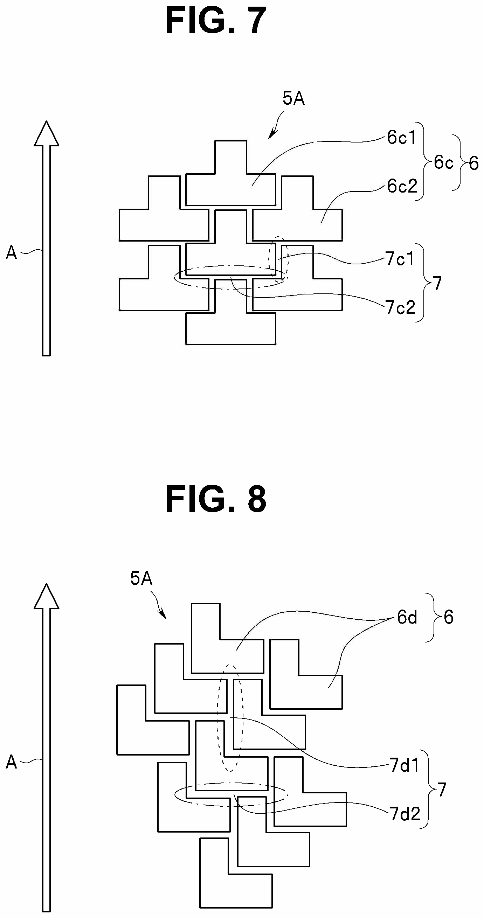

FIG. 6 and FIG. 7 show a third example of the related technology. FIG. 6 is a perspective view showing a flexible tube 5 in the third example of the related technology. FIG. 7 shows a configuration, in a planarly developed state, of an outer circumferential surface 5A of the flexible tube 5 in the third example of the related technology.

In the third example of the related technology, the same components as those in the first and second examples of the related technology are attached with the same reference signs and descriptions thereof will be omitted as appropriate. In the third example of the related technology, description will be made mainly on points different from the first and second examples of the related technology.

As shown in FIG. 6 and FIG. 7, each of a plurality of protruded surfaces 6 forms a T-shape 6c when the outer circumferential surface 5A of the flexible tube 5 is planarly developed. The T-shape 6c is a concave octagon (concave polygon) having two interior angles (specifically, interior angles of 270 degrees) which are reentrant angles (angles larger than 180 degrees and smaller than 360 degrees), and other six interior angles are 90 degrees.

The T-shape 6c is arranged with the long side (the longest side) being along the circumferential direction. In such an arrangement, the eight sides of the T-shape 6c are parallel to either the axial direction or the circumferential direction. Then, recessed surfaces 7 include first parts 7c1 along the axial direction (one first part 7c1 is shown by surrounding it by the dotted line in FIG. 7) and second parts 7c2 along the circumferential direction (one second part 7c2 is shown by surrounding it by the one-dot chain line in FIG. 7).

In other words, the recessed surfaces 7 each include at least one of the first part 7c1 or the second part 7c2. The first parts 7c1 are formed intermittently in the axial direction. Therefore, the length of the first parts 7c1 in the axial direction is shorter than the length of the entire flexible tube 5 in the axial direction. The second parts 7c2 are formed intermittently in the circumferential direction. Therefore, the length of the second parts 7c2 in the circumferential direction is shorter than the length of the outer circumferential surface 5A of the flexible tube 5 in the circumferential direction.

At this time, the depth of the first part 7c1 with respect to the protruded surface 6 may be made different from the depth of the second part 7c2 with respect to the protruded surface 6. In addition, the length of the first part 7c1 may be made different from the length of the second part 7c2. Furthermore, the width of the first part 7c1 may be made different from the width of the second part 7c2.

Among the plurality of T-shapes 6c, the two T-shapes 6c adjacent to each other in the circumferential direction are arranged such that arrangement pitches thereof in the axial direction are shifted by 1/2 pitches from each other. In other words, the plurality of T-shapes 6c include a T-shape 6c1 and a T-shape 6c2, the arrangement pitches thereof in the axial direction are shifted by 1/2 pitches from each other.

The third example of the related technology provides substantially the same effects as those of the first and second examples of the related technology.

In addition, according to the third example of the related technology, the first parts 7c1 along the axial direction and the second parts 7c2 along the circumferential direction are provided. With such a configuration, adjusting the depths, the lengths, and the widths of the first parts 7c1 and the second parts 7c2 enables the degree of freedom in controlling the characteristics to be enhanced.

### [Fourth Example of Related Technology]

FIG. 8 shows a configuration, in a planarly developed state, of an outer circumferential surface 5A of a flexible tube 5 in a fourth example of the related technology. In the fourth example of the related technology, the same components as those in the first to third examples of the related technology are attached with the same reference signs and descriptions thereof will be omitted as appropriate. In the fourth example of the related technology, description will be made mainly on points different from the first to third examples of the related technology.

As shown in FIG. 8, each of a plurality of protruded surfaces 6 forms an L-shape 6d, when the outer circumferential surface 5A of the flexible tube 5 is planarly developed. The L-shape 6d is a concave hexagon (concave polygon) having one interior angle (specifically, interior angle of 270 degrees) which is a reentrant angle, and other five interior angles are 90 degrees.

The L-shape 6d is arranged such that one of the two long sides is along the axial direction and the other is along the circumferential direction. In such an arrangement, the six sides of the L-shape 6d are parallel to either the axial direction or the circumferential direction. Then, recessed surfaces 7 include first parts 7d1 along the axial direction (one first part 7d1 is shown by surrounding it by the dotted line in FIG. 8) and second parts 7d2 along the circumferential direction (one second part 7d2 is shown by surrounding it by the one-dot chain line in FIG.8).

In other words, the recessed surfaces 7 each include at least one of the first part 7d1 or the second part 7d2. The first parts 7d1 are formed intermittently in the axial direction. Therefore, the length of the first parts 7d1 in the axial direction is shorter than the length of the entire flexible tube 5 in the axial direction. The second parts 7d2 are formed intermittently in the circumferential direction. Therefore, the length of the second parts 7d2 in the circumferential direction is shorter than the length of the outer circumferential surface 5A of the flexible tube 5 in the circumferential direction.

At this time, the depth of the first part 7d1 with respect to the protruded surface 6 may be made different from the depth of the second part 7d2 with respect to the protruded surface 6. In addition, the length of the first part 7d1 may be made different from the length of the second part 7d2. Furthermore, the width of the first part 7d1 may be made different from the width of the second part 7d2.

Note that FIG. 8 shows the L-shapes 6d each including the sides along the axial direction and the sides along the circumferential direction. This configuration does not have a plane symmetry to the plane including the center axis O. Therefore, each of the L-shapes 6d may be rotated, for example, by 45 degrees to be a V-shape, so as to create the plane symmetry to the plane including the center axis O.

The fourth example of the related technology provides substantially the same effects as those of the first to third examples of the related technology.

In addition, according to the fourth example of the related technology, the first parts 7d1 along the axial direction and the second parts 7d2 along the circumferential direction are provided. With such a configuration, adjusting the depths, the lengths, and the widths of the first parts 7d1 and the second parts 7d2 enables the degree of freedom in controlling the characteristics to be enhanced.

### [Fifth Example of Related Technology]

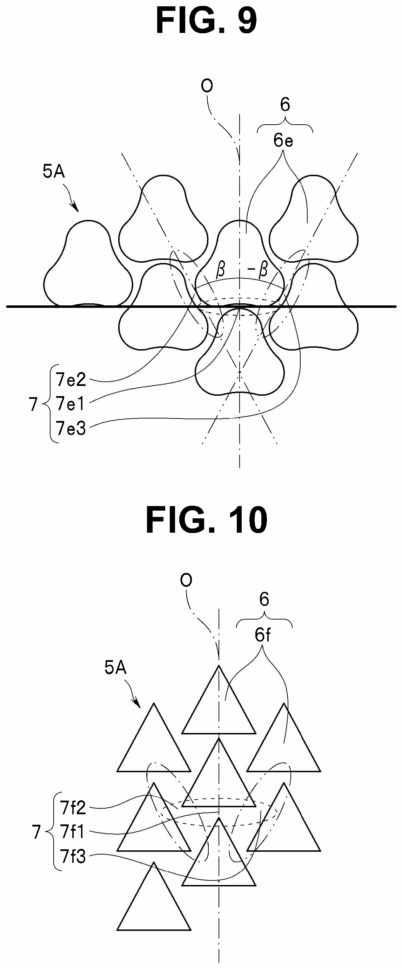

FIG. 9 shows a configuration, in a planarly developed state, of an outer circumferential surface 5A of a flexible tube 5 in a fifth example of the related technology. In the fifth example of the related technology, the same components as those in the first to fourth examples of the related technology are attached with the same reference signs and descriptions thereof will be omitted as appropriate. In the fifth example of the related technology, description will be made mainly on points different from the first to fourth examples of the related technology.

As shown in FIG. 9, each of a plurality of protruded surfaces 6 forms a rounded polygon when the outer circumferential surface 5A of the flexible tube 5 is planarly developed. The rounded polygon is roughly a shape that is formed by rounding corners of a polygon. In this case, the respective sides of the polygon are not limited to linear lines, and may be a concave arc toward the center of the rounded polygon, for example.

The example shown in FIG. 9 specifically shows rounded triangles 6e (for example, rounded equilateral triangles) each having three sides that form concave arcs. The rounded triangle 6e is arranged with a first side of the three sides being along the circumferential direction. In this arrangement, among the three sides of the rounded triangle 6e, a second side forms an angle β with respect to the axial direction and a third side forms an angle of - β with respect to the axial direction. When the rounded triangle 6e is a rounded equilateral triangle, for example, β is equal to 30 degrees (that is, 45 degrees or less).

Recessed surfaces 7 include first parts 7e1 along the first side (one first part 7e1 is shown by surrounding it by the dotted line in FIG. 9), second parts 7e2 along the second side (one second part 7e2 is shown by surrounding it by the one-dot chain line in FIG. 9), and third parts 7e3 along the third side (one third part 7e3 is shown by surrounding it by the two-dot chain line in FIG. 9).

The first parts 7e1 are formed intermittently in the circumferential direction. Therefore, the length of the first parts 7e1 in the circumferential direction is shorter than the length of the outer circumferential surface 5A of the flexible tube 5 in the circumferential direction. The second parts 7e2 and the third parts 7e3 are each formed intermittently in the axial direction and the circumferential direction. Therefore, the lengths of the second parts 7e2 and the third parts 7e3 in the axial direction are shorter than the length of the entire flexible tube 5 in the axial direction. In addition, angular ranges of the second parts 7e2 and the third parts 7e3 in the circumferential direction are smaller than 360 degrees.

At this time, the depths of the first part 7e1, the second part 7e2, and the third part 7e3 with respect to the protruded surface 6 may be made different from one another. In addition, the lengths of the first part 7e1, the second part 7e2, and the third part 7e3 may be made different from one another. Furthermore, the widths of the first part 7e1, the second part 7e2, and the third part 7e3 may be made different from one another.

The fifth example of the related technology provides substantially the same effects as those of the first to fourth examples of the related technology.

In addition, according to the fifth example of the related technology, the first parts 7e1, the second parts 7e2, and the third parts 7e3 that are oriented in the different directions are provided. With such a configuration, adjusting the depths, the lengths, and the widths of the first parts 7d1, the second parts 7e2, and the third parts 7e3 enables the degree of freedom in controlling the characteristics to be enhanced.

### [Sixth Example of Related Technology]

FIG. 10 shows a configuration, in a planarly developed state, of an outer circumferential surface 5A of a flexible tube 5 in a sixth example of the related technology. In the sixth example of the related technology, the same components as those in the first to fifth examples of the related technology are attached with the same reference signs and descriptions thereof will be omitted as appropriate. In the sixth example of the related technology, description will be made mainly on points different from the first to fifth examples of the related technology.

As shown in FIG. 10, each of a plurality of protruded surfaces 6 forms a polygon, when the outer circumferential surface 5A of the flexible tube 5 is planarly developed. The example shown in FIG. 10 specifically shows triangles 6f (for example, equilateral triangles).

In other words, the flexible tube 5 in the present example has a configuration using the triangles 6f, instead of the rounded triangles 6e in the fifth example of the related technology shown in FIG. 9.

The triangles 6f each include, for example, a first side along the circumferential direction, and a second side and a third side that intersect the axial direction (and the circumferential direction). When the triangle 6f is an equilateral triangle, for example, the second side and the third side intersect the axial direction at 30 degrees and -30 degrees, respectively.

Recessed surfaces 7 include first parts 7f1 along the first side (one first part 7f1 is shown by surrounding it by the dotted line in FIG. 10), second parts 7f2 along the second side (one second part 7f2 is shown by surrounding it by the one-dot chain line in FIG. 10), and third parts 7f3 along the third side (one third part 7f3 is shown by surrounding it by the two-dot chain line in FIG. 10).

The first parts 7f1 are formed intermittently in the circumferential direction. Therefore, the length of the first parts 7f1 in the circumferential direction is shorter than the length of the outer circumferential surface 5A of the flexible tube 5 in the circumferential direction. The second parts 7f2 and the third parts 7f3 are each formed intermittently in the axial direction and the circumferential direction. Therefore, the lengths of the second parts 7f2 and the third parts 7f3 in the axial direction are shorter than the length of the entire flexible tube 5 in the axial direction. In addition, angular ranges of the second parts 7f2 and the third parts 7f3 in the circumferential direction are smaller than 360 degrees.

At this time, the depths of the first part 7f1, the second part 7f2, and the third part 7f3 with respect to the protruded surface 6 may be made different from one another. In addition, the lengths of the first part 7f1, the second part 7f2, and the third part 7f3 may be made different from one another. Furthermore, the widths of the first part 7f1, the second part 7f2, and the third part 7f3 may be made different from one another.

The sixth example of the related technology provides substantially the same effects as those of the first to fifth examples of the related technology.

In addition, according to the sixth example of the related technology, the first parts 7f1, the second parts 7f2, and the third parts 7f3 that are oriented in the different directions are provided. With such a configuration, adjusting the depths, the lengths, and the widths of the first parts 7d1, the second parts 7f2, and the third parts 7f3 enables the degree of freedom in controlling the characteristics to be enhanced.

### [Seventh Example of Related Technology]

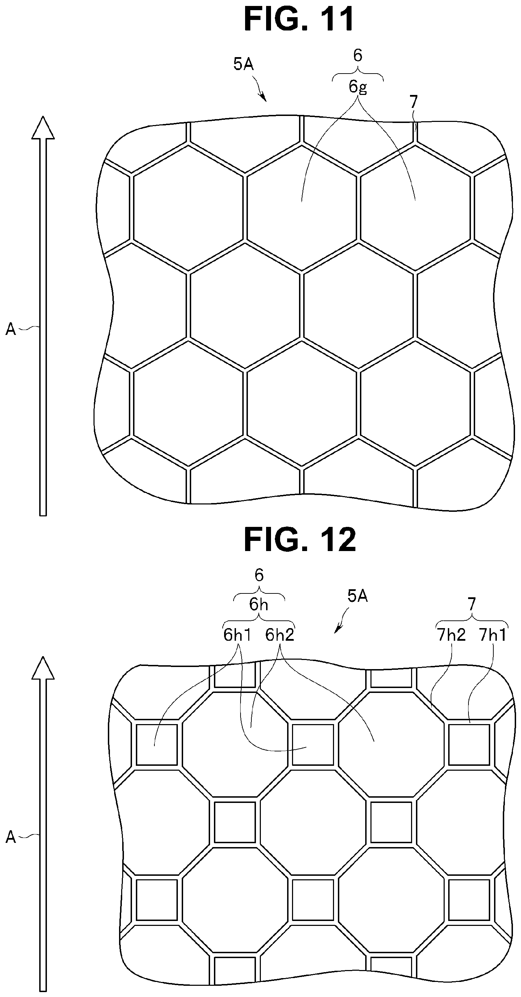

FIG. 11 shows a configuration, in a planarly developed state, of an outer circumferential surface 5A of a flexible tube 5 in a seventh example of the related technology. In the seventh example of the related technology, the same components as those in the first to sixth examples of the related technology are attached with the same reference signs and descriptions thereof will be omitted as appropriate. In the seventh example of the related technology, description will be made mainly on points different from the first to sixth examples of the related technology.

As shown in FIG. 11, each of a plurality of protruded surfaces 6 forms a polygon, when the outer circumferential surface 5A of the flexible tube 5 is planarly developed. The example shown in FIG. 10 specifically shows hexagons 6g (for example, regular hexagons).

The hexagons 6g shown in FIG. 11 are subjected to tiling such that, among three pairs of opposing sides of each of the hexagons 6g, a first pair of sides is parallel to the axial direction. When the hexagon 6g is a regular hexagon, for example, a second pair of sides and a third pair of sides intersect the axial direction at 60 degrees and -60 degrees, respectively.

Recessed surfaces 7 are provided between the hexagons 6g. At this time, the depths with respect to the protruded surfaces 6, the widths, and the like of the recessed surfaces 7 along the first pair of sides, the recessed surfaces 7 along the second pair of sides, and the recessed surfaces 7 along the third pair of sides may be made different from one another. In addition, the lengths of the respective recessed surfaces 7 may be made different from one another by employing the hexagons 6g other than the regular hexagons.

The seventh example of the related technology provides substantially the same effects as those of the first to sixth examples of the related technology.

According to the seventh example of the related technology, adjusting the depths, the lengths, and the widths of the recessed surfaces 7 according to the positions where the recessed surfaces 7 are provided enables the degree of freedom in controlling the characteristics to be enhanced.

### [Eighth Example of Related Technology]

FIG. 12 shows a configuration, in a planarly developed state, of an outer circumferential surface 5A of a flexible tube 5 in an eighth example of the related technology. In the eighth example of the related technology, the same components as those in the first to seventh examples of the related technology are attached with the same reference signs and descriptions thereof will be omitted as appropriate. In the eighth example of the related technology, description will be made mainly on points different from the first to seventh examples of the related technology.

In each of the first to seventh examples of the related technology, the plurality of protruded surfaces 6 respectively have the same shape. In contrast, in the present example, a configuration is employed in which tiling with protruded surfaces 6 is performed by combining the protruded surfaces 6 of two different types of shapes.

When the outer circumferential surface 5A of the flexible tube 5 is planarly developed, the plurality of protruded surfaces 6 include, for example, two types of polygons 6h, i.e., a quadrangle 6h1 (for example, square) and an octagon 6h2 (for example, regular octagon)

When the quadrangle 6h1 is a square and the octagon 6h2 is a regular octagon, the quadrangle 6h1 has four sides whose lengths are equal, the octagon 6h2 has eight sides whose lengths are equal, and the length of one side of the quadrangle 6h1 is equal to the length of one side of the octagon 6h2.

In the example shown in FIG. 12, the plurality of protruded surfaces 6 are arranged such that, of two pairs of sides of each of the quadrangles 6h1, a first pair of sides is in the axial direction and a second pair of sides is in the circumferential direction.

Recessed surfaces 7 include a first part 7h1 disposed between the quadrangle 6h1 and the octagon 6h2, and a second part 7h2 disposed between the octagons 6h2 adjacent to each other.

The depths, the lengths, and the widths of the first part 7h1 and the second part 7h2 may be made different according to the positions, directions (axial direction, circumferential direction, diagonal direction, etc.) and the like where the first part 7h1 and the second part 7h2 are disposed on the flexible tube 5.

The eighth example of the related technology provides substantially the same effects as those of the first to seventh examples of the related technology.

According to the eighth example of the related technology, adjusting the depths, the lengths, and the widths of the recessed surfaces 7 according to the positions and directions where the recessed surfaces 7 are provided enables the degree of freedom in controlling the characteristics to be enhanced.

Note that FIG. 12 shows the example in which the plurality of protruded surfaces 6 include two types of shapes, i.e., the quadrangle 6h1 and the octagon 6h2. However, another types of shapes may be combined. Furthermore, the shapes of the plurality of protruded surfaces 6 are not limited to two types, and may be three types or more.

### [Ninth Example of Related Technology]

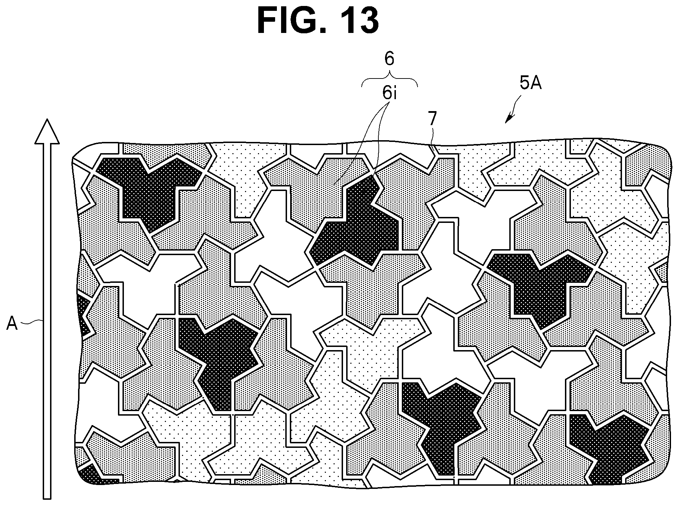

FIG. 13 shows a configuration, in a planarly developed state, of an outer circumferential surface 5A of a flexible tube 5 in a ninth example of the related technology. In the ninth example of the related technology, the same components as those in the first to eighth examples of the related technology are attached with the same reference signs and descriptions thereof will be omitted as appropriate. In the ninth example of the related technology, description will be made mainly on points different from the first to eighth examples of the related technology.

In the first to seventh examples of the related technology, the protruded surfaces 6 of the one type of shape are periodically aligned, and in the eighth example of the related technology, the protruded surfaces 6 of the two types of shapes are periodically aligned. In contrast, in the present example, a configuration is employed in which tiling with protruded surfaces 6 is performed by arranging the protruded surfaces of one type of shape aperiodically.

The plurality of protruded surfaces 6 are arranged aperiodically along the outer circumferential surface 5A. The protruded surfaces 6 shown in FIG. 13 are each a concave tridecagon (concave polygon) 6i having four interior angles which are reentrant angles. Note that, in FIG. 13, although different hatching patterns are used for easy discrimination among the plurality of concave tridecagons 6i, the plurality of concave tridecagons 6i have the same shape.

Specifically, the concave tridecagon 6i is also called as "Smith's hat", and recited in the website shown below, for example.

A chiral aperiodic monotile [David Smith1, Joseph Samuel Myers2, Craig S. Kaplan3, and Chaim Goodman-Strauss4] arXiv: 2305.17743v1 [math.CO] 28 May 2023 [retrieved on 2023-09-07] Retrieved from the Internet: (URL: https://arxiv.org/pdf/2305.17743.pdf)

In addition, recessed surfaces 7 are each arranged between the adjacent concave tridecagons 6i.

Note that the concave tridecagons 6i shown in FIG. 13 having the same shape can be arranged aperiodically. However, another example is also known in which even if the shapes are the same, both front and back sides can be combined and arranged aperiodically. Such an example is recited in the website shown below, for example. Such a configuration combining the front and back sides may be applied to the flexible tube 5.

An aperiodic monotile [David Smith1, Joseph Samuel Myers*2, Craig S. Kaplan3, and Chaim Goodman-Strauss4] arXiv:2303.10798v2 [math. CO] 29 May 2023 [retrieved on 2023-09-07] Retrieved from the Internet: (URL: https://arxiv.org/pdf/2303.10798.pdf)

Furthermore, an example called penrose tiling is also known in which figures are arranged aperiodically by combining two types of shapes. Such a configuration combining a plurality of types of shapes may be applied to the flexible tube 5.

The ninth example of the related technology provides substantially the same effects as those of the first to eighth examples of the related technology.

According to the ninth example of the related technology, it is possible to obtain a characteristic which is substantially even from a broad perspective but uneven from a local perspective.

### [Tenth Example of Related Technology]

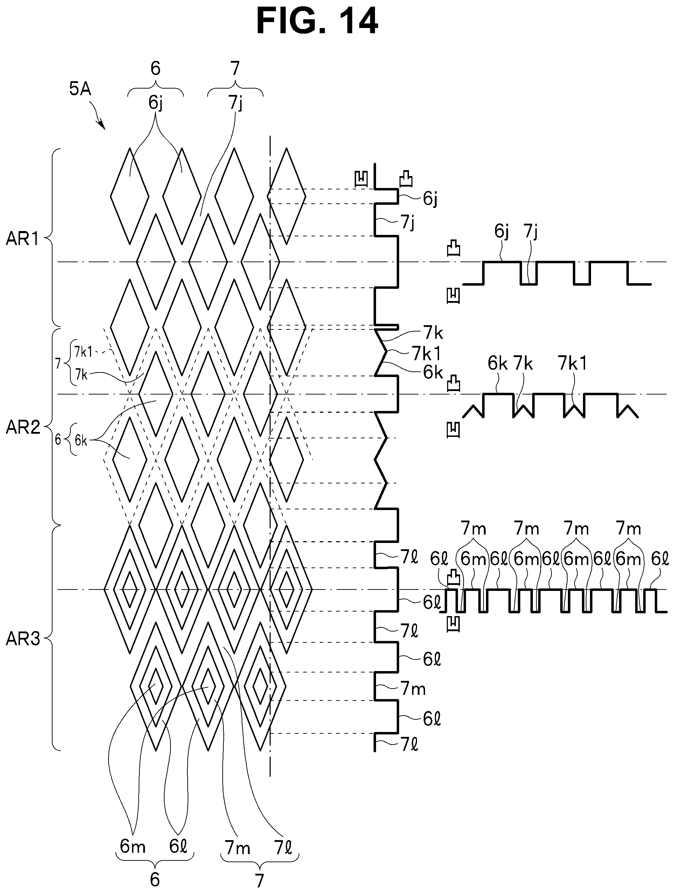

FIG. 14 shows a configuration, in a planarly developed state, of an outer circumferential surface 5A of a flexible tube 5 in a tenth example of the related technology. In the tenth example of the related technology, the same components as those in the first to ninth examples of the related technology are attached with the same reference signs and descriptions thereof will be omitted as appropriate. In the tenth example of the related technology, description will be made mainly on points different from the first to ninth examples of the related technology.

In the first to ninth examples of the related technology, the two-dimensional shape of the protruded surfaces 6 (further, the recessed surfaces 7) is constant, in principle, regardless of the positions thereof in the axial direction. In contrast, in the present example, the two-dimensional shape of protruded surfaces 6 (further, recessed surfaces 7) is changed along the axial direction.

As shown in FIG. 14, the flexible tube 5 includes a first area AR1, a second area AR2, and a third area AR3 along the axial direction.

Note that FIG. 14 shows also a chart showing surface shapes in the cases where the outer circumferential surface 5A of the flexible tube 5, which has been planarly developed, is cut respectively along four one-dot chain lines (a one-dot chain line in the axial direction, a one-dot chain line in the circumferential direction in the first area AR1, a one-dot chain line in the circumferential direction in the second area AR2, and a one-dot chain line in the circumferential direction in the third area AR3).

The first area AR1 is an area where the bendability is relatively low (that is, rigid), for example. Specifically, each of the plurality of protruded surfaces 6 aligned in the first area AR1 forms a rhombus 6j, when the outer circumferential surface 5A of the flexible tube 5 is planarly developed. The recessed surfaces 7j are each provided between the adjacent rhombuses 6j.

The second area AR2 is an area where the bendability is relatively medium (that is, middle rigidity), for example. Specifically, each of a plurality of protruded surfaces 6 aligned in the second area AR2 forms a rhombus 6k, when the outer circumferential surface 5A of the flexible tube 5 is planarly developed. An area of one rhombus 6k is smaller than an area of one rhombus 6j, for example.

Recessed surfaces 7k are each provided between the adjacent rhombuses 6k. The recessed surface 7k includes, at the center thereof, a chevron 7k1. A height of the chevron 7k1 is lower than a height of the rhombus 6k. In other words, also the chevron 7k1 is a part of the recessed surface 7k when viewed from the rhombus 6k which is the protruded surface 6. The chevron 7k1 is provided, which enables the recessed surface 7k to be bent easily, and increases the extension/contraction property of the recessed surface 7k in the direction orthogonal to the chevron 7k1.

A ratio of the area of the protruded surfaces 6 to the area of the recessed surfaces 7 for each of the areas is larger in the first area AR1 than in the second area AR2, for example.

The third area AR3 is an area where the bendability is relatively high (that is, soft), for example. Comparison among the bendabilities in the respective areas AR1 to AR3 provides the following. In the axial direction and the circumferential direction, the number of protrusions and recesses per unit length is larger in the second area AR2 than in the first area AR1, and larger in the third area AR3 than in the second area AR2. With such a configuration, the bendability is higher in the second area AR2 than in the first area AR1, and higher in the third area AR3 than in the second area AR2.

The plurality of protruded surfaces 6 aligned in the third area AR3 each include a first protruded surface 6l and an inner rhombus 6m, when the outer circumferential surface 5A of the flexible tube 5 is planarly developed.

The first protruded surfaces 6l are protruded surfaces formed by connecting a plurality of ring-shaped rhombuses (hereinafter, referred to as outer rhombuses) in the circumferential direction. It is understood that the plurality of outer rhombuses are connected in the circumferential direction from the chart showing the surface shape when the outer circumferential surface 5A is cut along the one-dot chain line in the circumferential direction in the third area AR3. However, the plurality of outer rhombuses may be isolated from one another, instead of connecting them in the circumferential direction.

The inner rhombuses 6m are protruded surfaces each arranged in the inside of each of the plurality of outer rhombuses of the first protruded surfaces 61, spaced apart from each of the plurality of outer rhombuses without being connected thereto. Thus, inside a given protruded surface 6, another protruded surface 6 may be provided.

Recessed surfaces 7l are each provided between the first protruded surfaces 6l adjacent to each other in the axial direction. In addition, recessed surfaces 7m are each provided between the outer rhombus of the first protruded surface 6l and the inner rhombus 6m. Thus, the first protruded surface 6l and the inner rhombus 6m are each surrounded by the recessed surface 7 (recessed surface 7l or the recessed surface 7m) and isolated from another protruded surface 6.

Accordingly, each of the plurality of protruded surfaces 6 has a first shape in a first range (for example, any one area of the first area AR1, the second area AR2, and the third area AR3) along the axial direction. In addition, each of the plurality of protruded surfaces 6 has a second shape different from the first shape in a second range (for example, another one area of the first area AR1, the second area AR2, and the third area AR3) along the axial direction, which is different from the first range.

Note that, in the above-described example, the shape patterns of the protruded surfaces 6 and recessed surfaces 7 are changed for each of the areas, but are not limited to such an example. For example, morphing may be used to configure the shape patterns of the protruded surfaces 6 and recessed surfaces 7 change smoothly along the axial direction.

The tenth example of the related technology provides substantially the same effects as those of the first to ninth examples of the related technology.

According to the tenth example of the related technology, the shape patterns of the protruded surfaces 6 and the recessed surfaces 7 are changed in the axial direction. With such a configuration, depending on how to change the shape patterns, the characteristics such as the bendability, the extension/contraction resistance, and the torque resistance in the axial direction can be controlled at a higher degree of freedom in the axial direction. Such a configuration enables the one flexible tube 5 to have both the area suitable for the tubular portion 2c and the area suitable for the bending portion 2b, for example.

### [Eleventh Example of Related Technology]

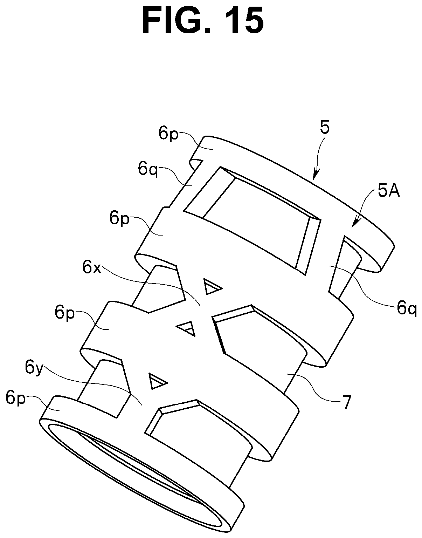

FIG. 15 is a perspective view showing a flexible tube 5 in an eleventh example of the related technology. In the eleventh example of the related technology, the same components as those in the first to tenth examples of the related technology are attached with the same reference signs and descriptions thereof will be omitted as appropriate. In the eleventh example of the related technology, description will be made mainly on points different from the first to tenth examples of the related technology.

The flexible tube 5 in each of the first to tenth examples of the related technology includes the plurality of protruded surfaces 6 each surrounded by the recessed surfaces 7 to be isolated from one another. In contrast, the flexible tube 5 in the present example is formed by connecting a plurality of protruded surfaces 6 each forming a ring shape in the circumferential direction to one another in the axial direction.

The flexible tube 5 can be applied to the insertion portion 2 including the bending portion 2b and the tubular portion 2c. If a corrugated tube having a bellows structure is used for the tubular portion 2c, for example, it can contribute to an improvement of the procedure (excellent bending performance, reduction of fatigue of an operator) due to a light weight and an excellent bendability of such a corrugated tube.

However, the corrugated tube extends and contracts in the axial direction. Therefore, if the corrugated tube is used for the insertion portion 2, when a push/pull operation is performed in a procedure using a lower gastrointestinal endoscope, for example, a difference occurs between a sense of insertion of an operator and an actual insertion length, which results in a degradation of the operability in the procedure. In view of the above, a configuration of the flexible tube 5 whose extension/contraction resistance is improved than that of the corrugated tube will be described with reference to FIG. 15.

The flexible tube 5 shown in FIG. 15 includes a plurality of protruded surfaces 6p each forming a ring shape in the circumferential direction. Recessed surfaces 7 are each arranged between the protruded surfaces 6p adjacent to each other. Note that filling structure parts 9 may be formed respectively in the recessed surfaces 7 by filling the recessed surfaces 7 with a filling material, similarly to the above-described embodiments.

The two adjacent protruded surfaces 6p are connected with a connecting protruded surface. The two adjacent protruded surfaces 6p are relatively hard to extend and contract at the part where the connecting protruded surface is provided, and relatively easy to extend and contract at the part where the connecting protruded surface is not provided.

For example, the two adjacent protruded surfaces 6p are connected to each other by using a plurality of connecting protruded surfaces 6q that are inclined with respect to the axial direction (that is, not parallel to the axial direction). For example, the two connecting protruded surfaces 6q are inclined at respective angles symmetrical to the axial direction.

In a first case shown in FIG. 15, the plurality of connecting protruded surfaces 6q are arranged apart from one another in the circumferential direction. The connecting protruded surfaces 6q are connected to each of the two protruded surfaces 6p at a specific angle.

Note that when only the two connecting protruded surfaces 6q are arranged at the positions shown in FIG. 15, the two connecting protruded surfaces 6q are not arranged at the 180-degree opposite positions in the circumferential direction. Arranging the connecting protruded surfaces 6q at uneven and deviated positions around the center axis O enables control of a direction in which bending is easy and a direction in which bending is hard.

In addition, in a second case shown in FIG. 15, the two adjacent protruded surfaces 6p are connected to each other by using a connecting protruded surface 6x formed in an X-shape, for example. The connecting protruded surface 6x is configured such that a plurality of (two in the case shown in the drawing) protruded surfaces 6 are arranged so as to intersect each other. Note that the position where the plurality of protruded surfaces 6 intersect each other is not limited to the center position of the connecting protruded surface 6x (center position of the connecting protruded surface 6x in the axial direction). Also in this case, the connecting protruded surface 6x is connected to each of the two protruded surfaces 6p at a specific angle.

Furthermore, in a third case shown in FIG. 15, the two adjacent protruded surfaces 6p are connected to each other by using a connecting protruded surface 6y formed in a Y-shape, for example. The connecting protruded surface 6y is formed such that a plurality of protruded surfaces 6 are merged at a halfway point, to form one protruded surface. Note that the position where the plurality of protruded surfaces 6 are merged is not limited to the center position of the connecting protruded surface 6y. In this case, the connecting protruded surface 6y is configured such that two branched parts are connected to one of the protruded surfaces 6p at a specific angle. In addition, the merged part of the connecting protruded surface 6y is connected, for example, orthogonally to the other of the protruded surfaces 6p.

Note that FIG. 15 shows the example in which the three types of connecting protruded surfaces 6q, 6x, and 6y are provided. However, needless to say, only any one or two types of the connecting protruded surfaces may be provided.

According to the eleventh example of the related technology, the connecting protruded surfaces 6q, 6x, and 6y are provided, which can suppress the extension/contraction characteristic of the corrugated tube in the axial direction. Such a configuration can provide the flexible tube 5 which is suitable for the bending portion 2b and the tubular portion 2c that require the extension/contraction resistance.

In addition, the connecting protruded surfaces 6q, 6x, and 6y each have the part inclined with respect to the axial direction, which can provide the extension/contraction resistance and the torque resistance when a rotation torque around the center axis O is applied to the flexible tube 5. At this time, adjusting the inclined angles enables adjustment of the balance between the extension/contraction resistance and the torque resistance of the flexible tube 5.

Furthermore, adjusting the positions of the connecting protruded surfaces 6q, 6x, and 6y in the circumferential direction enables control of the direction in which the flexible tube 5 is easy to bend and the direction in which the flexible tube 5 is hard to bend. On the other hand, equalizing the positions of the protruded surfaces to be connected in the circumferential direction can equalize the direction in which the flexible tube 5 is easy to bend.

In addition, the connecting protruded surfaces 6q, 6x, and 6y, the positions of which in the axial direction are different from one another, are arranged at different positions in the circumferential direction, which can equalize the easiness of the extension/contraction of the flexible tube.

Furthermore, since the connecting protruded surface 6x formed in the X-shape and the connecting protruded surface 6y formed in the Y-shape each have the branched part, when a stress is applied to the flexible tube 5, the stress acts separately thereon. As a result, the stress that acts on the flexible tube 5 is relieved, which can suppress the extension and contraction of the flexible tube 5.

### [Twelfth Example of Related Technology]

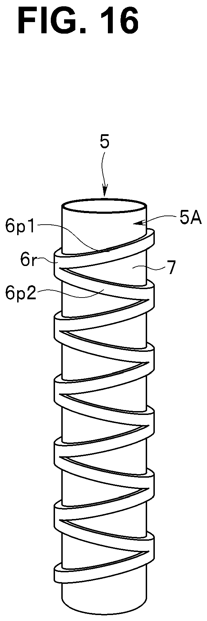

FIG. 16 is a perspective view showing a flexible tube 5 in a twelfth example of the related technology. In the twelfth example of the related technology, the same components as those in the first to eleventh examples of the related technology are attached with the same reference signs and descriptions thereof will be omitted as appropriate. In the twelfth example of the related technology, description will be made mainly on points different from the first to eleventh examples of the related technology.

The flexible tube 5 in the present example is formed by arranging a plurality of protruded surfaces 6, each of which forms a ring shape in the circumferential direction, so as to be inclined at angles alternately different with respect to the circumferential direction and by connecting the plurality of protruded surfaces 6 at the parts where they are proximity to each other.

The flexible tube 5 includes ring-shaped first protruded surfaces 6p1 inclined at a first angle with respect to the circumferential direction and ring-shaped second protruded surfaces 6p2 inclined at a second angle with respect to the circumferential direction. Each of the first protruded surfaces 6p1 and each of the second protruded surfaces 6p2 are connected to each other by a connecting portion 6r. The connecting portion 6r is a protruded surface on which each of the first protruded surface 6p1 and each of the second protruded surface 6p2 intersect each other.

In other words, when focusing on a given first protruded surface 6p1, the given first protruded surface 6p1 is connected to the second protruded surface 6p2 which is on one side with respect to the axial direction by a first connecting portion 6r, and connected to the second protruded surface 6p2 which is on the other side with respect to the axial direction by a second connecting portion 6r. The first connecting portion 6r and the second connecting portion 6r are located, for example, at opposite positions in the circumferential direction, that is, at the positions different from each other by 180 degrees in the circumferential direction.

For example, if the angle in the circumferential direction at which the first connecting portion 6r is provided is supposed to be 0 degrees and the angle in the circumferential direction at which the second connecting portion 6r is provided is supposed to be 180 degrees, no connecting portion 6r is present at a position between 0 to 180 degrees, and a position between 180 to 0 degrees.

Note that, although the example in which the connecting portions 6r are provided at the positions different from each other by 180 degrees in the circumferential direction is shown here, adjusting the angles in the circumferential direction at which the connecting portions 6r are provided enables control of a direction in which bending is easy and a direction in which bending is hard.

Thus, the first protruded surface 6p1 is connected to the two second protruded surfaces 6p2 that are adjacent in the axial direction at one location each, i.e., at two locations in total. In addition, the second protruded surface 6p2 is connected to the two first protruded surfaces 6p1 that are adjacent in the axial direction at one location each, i.e., at two locations in total.

Recessed surfaces 7 are each provided between each of the first protruded surfaces 6p1 and each of the second protruded surfaces 6p2, except for the connecting portions 6r. Note that filling structure parts 9 may be provided respectively in the recessed surfaces 7 by filling the recessed surfaces 7 with a filling material, similarly to the above-described embodiments.

The twelfth example of the related technology provides substantially the same effects as those of the eleventh examples of the related technology. In addition, adjusting the inclined angles of the first protruded surfaces 6p1 and the second protruded surfaces 6p2 enables the adjustment of the balance between the extension/contraction resistance and the torque resistance of the flexible tube 5.

### [Thirteenth Example of Related Technology]

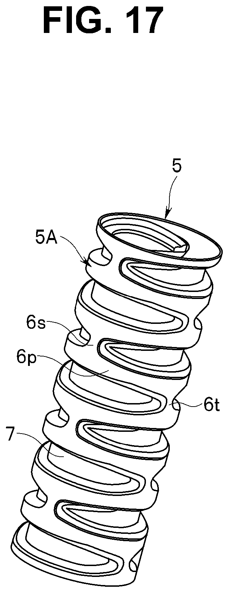

FIG. 17 is a perspective view showing a flexible tube 5 in a thirteenth example of the related technology. In the thirteenth example of the related technology, the same components as those in the first to twelfth examples of the related technology are attached with the same reference signs and descriptions thereof will be omitted as appropriate. In the thirteenth example of the related technology, description will be made mainly on points different from the first to twelfth examples of the related technology.

The flexible tube 5 in the present example is formed by connecting two protruded surfaces 6, each of which forms a ring shape in the circumferential direction, at a plurality of locations in the circumferential direction.

The flexible tube 5 includes the plurality of protruded surfaces 6p, each of which forms the ring shape in the circumferential direction.

When focusing on a given protruded surface 6p, the given protruded surface 6p is connected to the protruded surface 6p which is on one side with respect to the axial direction at a first connecting portion 6s, and connected to the protruded surface 6p which is on the other side with respect to the axial direction at a second connecting portion 6t. Both the first connecting portion 6s and the second connecting portion 6t are the protruded surfaces 6.

The first connecting portions 6s are provided respectively at two locations different from each other by 180 degrees in the circumferential direction, for example. The second connecting portions 6t are provided respectively at two locations different from each other by 180 degrees in the circumferential direction, for example. The first connecting portions 6s and the second connecting portions 6t differ in the angle around the center axis O by 90 degrees, for example.

With such a configuration, a given protruded surface 6p is connected to the protruded surface 6p which is on the one side with respect to the axial direction by the first connecting portions 6s at the two locations and connected to the protruded surface 6p which is on the other side with respect to the axial direction by the second connecting portions 6t at the two locations. Note that the first connecting portions 6s and the second connecting portions 6t may be provided so as to be inclined with respect to the axial direction or parallel to the axial direction.

Here, a given protruded surface 6p may have a wave-shaped inclination along the circumferential direction such that the given protruded surface 6p is in proximity to the protruded surface 6p on one side at the first connecting portions 6s at the two locations and is in proximity to the protruded surface 6p on the other side at the second connecting portions 6t at the two locations.

Recessed surfaces 7 are each provided between the plurality of protruded surfaces 6p, except for the connecting portions 6s and 6t. Note that filling structure parts 9 may be provided respectively in the recessed surfaces 7 by filling the recessed surfaces 7 with a filling material, similarly to the above-described embodiments.

The thirteenth example of the related technology provides substantially the same effects as those of the above-described twelfth example of the related technology.

Note that FIG. 16 of the twelfth example of the related technology shows the example in which the given protruded surface 6p is connected to the other protruded surfaces 6p at each of the upper one location and the lower one location (two locations in total). In addition, FIG. 17 of the thirteenth example of the related technology shows the example in which the given protruded surface 6p is connected to the other protruded surfaces 6p at each of the upper two locations and the lower two locations (four locations in total). The configurations are not limited to these examples, and a configuration may be employed in which a given protruded surface 6p is connected to other protruded surfaces 6p at three locations or more in total.

### [First Embodiment]

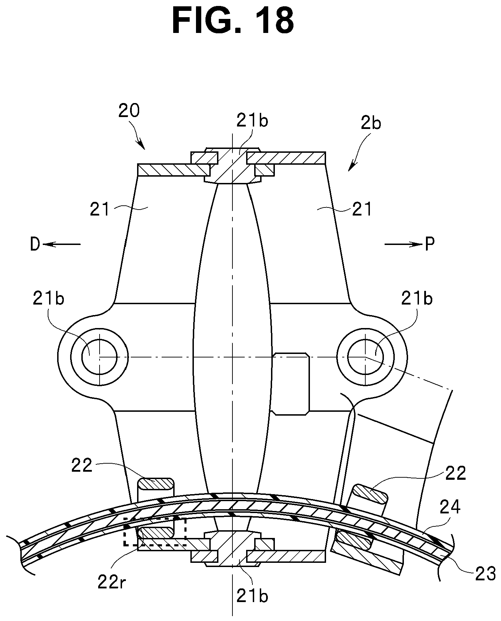

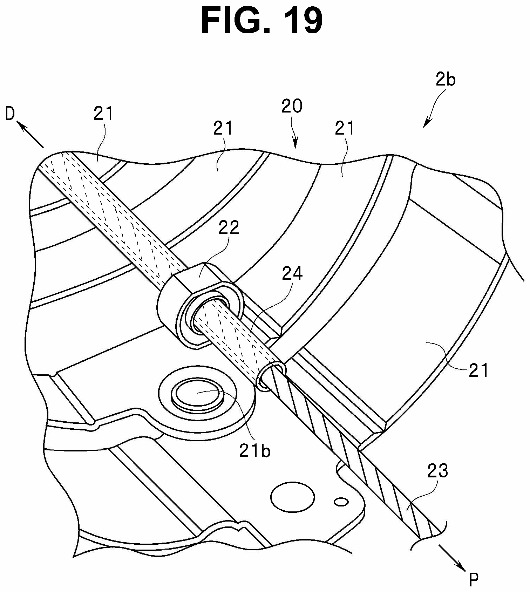

FIG. 18 to FIG. 22 show a first embodiment of the present disclosure. FIG. 18 is a cross-sectional view showing a configuration example of a bending wire 23 in a bending portion 2b in the first embodiment. FIG. 19 is a partial perspective view showing the configuration example of the bending wire 23 in the bending portion 2b in the first embodiment.

Note that, in FIG. 18 and subsequent respective drawings, the proximal end direction (direction of the operation portion 3 side) of the entire endoscope 1 is indicated by the arrow P, and the distal end direction (direction of the distal end portion 2a side) of the entire endoscope 1 is indicated by the arrow D.

In addition, in FIG. 18 and subsequent respective drawings, the bending portion denoted by the reference sign 2b and a tubular portion denoted by the reference sign 2c are shown in a state where exterior members such as a braid and an outer cover are removed, in order to clearly show the internal configurations thereof. The bending portion 2b and the tubular portion 2c are configured actually in the state where the outer surfaces thereof are covered with the braid and the outer cover.

Furthermore, inside the bending portion 2b and the tubular portion 2c, in addition to the bending wire 23 inserted into a coil pipe 25, internal components such as a gas/liquid feeding conduit and a treatment instrument insertion channel are inserted. However, since these internal components are not directly related to the present disclosure, illustrations thereof are omitted.

In the first embodiment, the same components as those in the respective examples of the above-described related technology are attached with the same reference signs and descriptions thereof will be omitted as appropriate. In the first embodiment, description will be made mainly on points different from the respective examples of the related technology.

The bending portion 2b (first tube) is formed along an axis (center axis O: see FIG. 21) extending from the proximal end direction P side (first side) to the distal end direction D side (second side). The bending portion 2b is configured to be bent by the bending wire 23 (wire) being pulled.

In other words, the bending wire 23 is inserted, from the bending portion 2b to the tubular portion 2c, and the proximal end direction P side (first side) of the bending wire 23 is connected to a drum and the like in the operation portion 3. The drum in the operation portion 3 rotates in conjunction with the bending operation knob 3b.

When the bending operation knob 3b of the operation portion 3 is operated, the bending wire 23, which is arranged inside from the operation portion 3 to an insertion portion 2, is pulled, and the bending portion 2b is bent. Note that the bending wire 23 includes four wires, for example, in an endoscope provided with a bending portion 2b configured to be bendable in four directions, i.e., up, down, left and right directions, and the bending wire 23 includes two wires, for example, in an endoscope provided with a bending portion 2b configured to be bendable in two directions, i.e., up and down directions. Hereinafter, description will be made by taking the endoscope 1 configured to be bendable in four directions as an example.

The bending portion 2b (first tube) includes a plurality of bending pieces 21 (a plurality of cylinders) and a connecting mechanism 21b such as hinges. The plurality of bending pieces 21 constituting the bending portion 2b are made of metal, for example. The plurality of bending pieces 21 are aligned along the center axis O (axis).

The connecting mechanism 21b connects two adjacent bending pieces 21 among the plurality of bending pieces 21 such that one of the bending pieces 21 (cylinder) can deflect with respect to the other of the bending piece 21 (cylinder). Thus, the plurality of bending pieces 21 are swingably connected to one another with the connecting mechanism 21b.

FIG. 18, FIG. 21, etc., show an example of the bending portion 2b configured to be bendable in the four directions, i.e., up, down, left and right directions, and the bending portion 2b includes the connecting mechanism 21b for bending in the up and down directions and the connecting mechanism 21b for bending in the left and right directions.

The distal end (end on the second side) of the bending wire 23 is fixed to the distal end side of the bending portion 2b, and fixed, for example, to the bending piece 21a arranged at the distal-most position at a fixing portion 24f (see FIG. 21, etc.)

In the bending portion 2b, wire receivers 22 are fixed to some of the plurality of bending pieces 21. The plurality of wire receivers 22 are arranged at appropriate intervals along the axial direction of the bending portion 2b. The wire receivers 22 each form a ring shape (see FIG. 19), for example, and the bending wire 23 is inserted therethrough.

The bending mechanism 20 includes the bending portion 2b (first tube), tubes 24 (first guides), wire receivers 22 (second guides) that will be described later, and further includes the bending wires 23 (wires). The tubes 24 are formed to be more flexible than the wire receivers 22, for example.

As described above, the proximal end side of the bending wire 23 is arranged in the operation portion 3. Therefore, at least a part (a part except for the part of the bending wires 23, which are arranged in the operation portion 3 and on the first side (proximal end direction P side) of the insertion portion 2) of the bending mechanism 20 is provided on the second side (distal end direction D side) of the insertion portion 2.

The connecting mechanism 21b is formed separately from the tubes 24 (first guide). Therefore, the connecting mechanism 21b and the tubes 24 may be made of different materials.

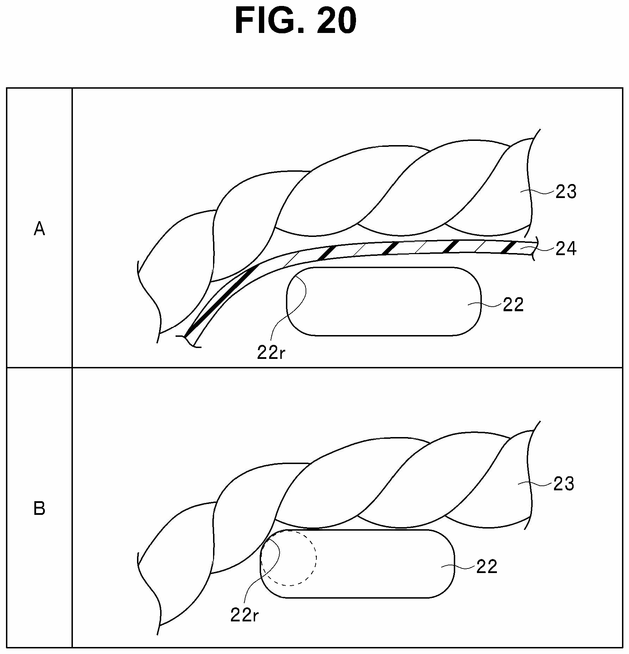

FIG. 20 is a chart showing a comparison of a relationship between the bending wire 23 inserted through the tube 24 and the wire receiver 22 in the first embodiment, and a relationship between a conventional bending wire 23 and a wire receiver 22. In FIG. 20, the column A shows the configuration of the present embodiment and the column B shows the conventional configuration.

As shown in FIG. 20, the bending wire 23 is configured of a twisted wire, for example, and has, on the surface thereof, an unevenness. The wire receiver 22 has a corner portion 22r formed by being rounded in an R-shape as shown in the column B in FIG. 20, in order to prevent the unevenness on the surface of the bending wire 23 from being caught.

Incidentally, the bending portion 2b includes a type configured such that a maximum bending angle exceeds 180 degrees. In such a type, when the insertion portion 2 of the endoscope 1 is inserted into a subject, there is a case where the bending portion 2b of the insertion portion 2 becomes a complicated shape such as a loop shape.

When a bending operation is performed to pull the bending wire 23 in the state where the bending portion 2b is in the complicated shape, even if the corner portion 22r of the wire receiver 22 is formed in the R-shape, friction and catching occur between the unevenness on the surface of the bending wire 23 and the inner circumferential surface and the corner portion 22r of the wire receiver 22, which results in an increase in a sliding resistance.

In such a case, a loss occurs in the pulling force of the bending wire 23 by the wire receiver 22, and an amount of the operation force required for operating the bending operation knob 3b increases according to the sliding resistance, which results in a deterioration in the operability. In addition, abrasion, scraping, and the like occur in the corner portion 22r of the wire receiver 22.

In order to address such a problem, as shown in FIG. 18, FIG. 19, and the column A in FIG. 20, the bending wire 23 in the first embodiment, in a state of being inserted through the tube 24, is inserted through the wire receiver 22. The tube 24 is the first guide for holding the bending wire 23. The wire receiver 22 is the second guide for holding the tube 24.

The tube 24 is made of a resin such as HDPE (high-density polyethylene) or PTFE (polytetrafluoroethylene) and formed in a cylindrical shape as a tube which has a surface with good sliding property, and which is unlikely to wrinkle. Furthermore, the tube 24 has a thickness set such that a wrinkle hardly occurs on the inner circumferential side when the tube 24 is bent. The friction coefficient between the bending wire 23 and the tube 24 is smaller than the friction coefficient between the bending wire 23 and the wire receiver 22.

FIG. 21 is a cross-sectional view showing a configuration of the tubes 24 through which the bending wires 23 are respectively inserted, in the bending portion 2b in a straight state, in the first embodiment.

The tubes 24 are each provided in the bending portion 2b along the center axis O shown in FIG. 21. The distal end of each of the tubes 24 is, together with the distal end of the bending wire 23, fixed to the bending piece 21a at the fixing portion 24f. On the other hand, the proximal end of each of the tubes 24 is not fixed. Thus, regarding the movement in the axial direction (direction of the center axis O), the distal end of each of the tubes 24 is a fixed end, while the proximal end thereof is a free end.

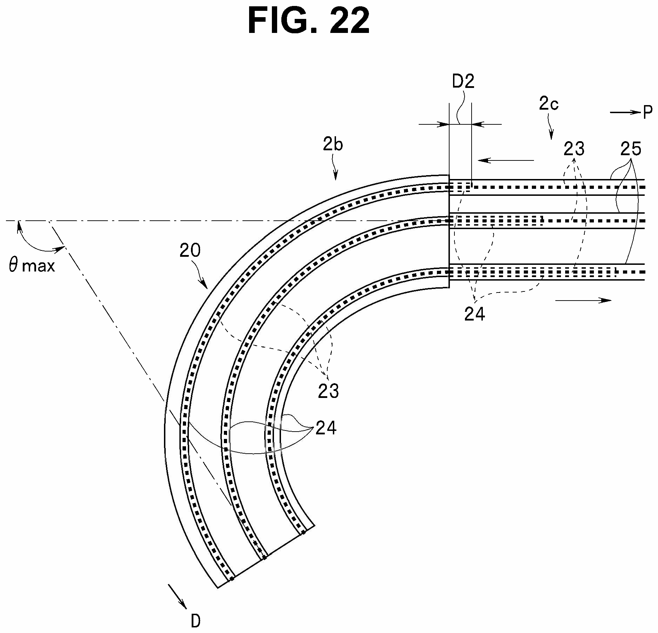

FIG. 22 is a cross-sectional view showing a state of the tubes 24 through which the bending wires 23 are respectively inserted, in the bending portion 2b in a maximum bending state, in the first embodiment.

The tubular portion 2c (second tube) is provided, along the center axis O, on the proximal end side (proximal end direction P side) relative to the bending portion 2b. In the tubular portion 2c, the coil pipes 25 are arranged along the center axis O. The coil pipes 25 are each a third guide arranged along the center axis O in the tubular portion 2c. The coil pipes 25 are fixed to a bonding part (not shown) between the bending portion 2b and the tubular portion 2c.

Each of the tubes 24, the distal end of which is fixed at the fixing portion 24f, is inserted through the plurality of wire receivers 22, and further inserted through the proximal-most wire receiver 22. After that, when each of the tubes 24 reaches the tubular portion 2c, each of the tubes 24 is arranged to enter the coil pipe 25 up to a halfway position thereof. FIG. 21 shows that the tubes 24 are provided such that the entered length of each of the tubes 24 respectively into the coil pipes 25, is a first predetermined length D3, when the bending portion 2b is in the straight state.

When each of the bending wires 23 is pulled, the distal end of each of the tubes 24, which is the fixed end, does not move in the axial direction, and the proximal end of each of the tubes 24, which is the free end, moves in the axial direction. For example, the proximal end of the tube 24, which is located on the outer circumferential side when the bending portion 2b is bent, moves toward the distal end side (distal end direction D side) and the proximal end of the tube 24, which is located on the inner circumferential side when the bending portion 2b is bent, moves toward the proximal end side (proximal end direction P side).

FIG. 22 shows that, when the bending portion 2b is in the maximum bending state, in the tube 24 extended most toward the distal end side, the entered length of the tube 24 into the coil pipe 25 is a second predetermined length D2.

In a specific product, the second predetermined length D2 may be set to 1 mm or more and 20 mm or less, for example. In addition, the predetermined second length D2 may be set to 10 mm or more, for example. However, the second predetermined length D2 is not limited to these numerical values.

The first predetermined length D3 is set to such a length (length ensuring the second predetermined length D2) that the proximal end of the tube 24 (the same is true on all the tubes 24, but in particular, the tube 24 which is on the outer circumferential side at the time of bending) does not fall off (come out) from the distal end of the coil pipe 25, even in the state the bending portion 2b is maximally bent.

Here, the case is supposed in which four bending wires 23 are provided respectively for bending in R (right), L (left), U (up), and D (down) directions.

When the bending wire 23 for R (right) bending is pulled maximally, the bending portion 2b is brought into the maximum bending state in the R (right) direction. At this time, the tube 24 through which the bending wire 23 for L (left) bending is inserted is extended most toward the distal end side.

When the bending wire 23 for L (left) bending is pulled maximally, the bending portion 2b is brought into the maximum bending state in the L (left) direction. At this time, the tube 24 through which the bending wire 23 for R (right) bending is inserted is extended most toward the distal end side.

When the bending wire 23 for U (up) bending is pulled maximally, the bending portion 2b is brought into the maximum bending state in the U (up) direction. At this time, the tube 24 through which the bending wire 23 for D (down) bending is inserted is extended most toward the distal end side.

When the bending wire 23 for D (down) bending is pulled maximally, the bending portion 2b is brought into the maximum bending state in the D (down) direction. At this time, the tube 24 through which the bending wire 23 for U (up) bending is inserted is extended most toward the distal end side.

The first predetermined length D3 is set based on a length R (see FIG. 21) in the radial direction, which is from the center axis O of the bending portion 2b to the center of the wire receiver 22, a length L (see FIG. 21) of the bending portion 2b in the direction of the center axis O, and a bending angle θ max (see FIG. 22) when the bending portion 2b is maximally bent.

The bending angle θ max when the bending portion 2b is maximally bent differs in some cases depending on whether the bending direction is the R (right), the L (left), the U (up) or the D (down) direction. Accordingly, even if the second predetermined lengths D2 according to the R (right), the L (left), the U (up) or the D (down) direction at the time of maximum bending are set to the same value, the first predetermined lengths D3 differ in some cases depending on whether the tube 24 includes, inside thereof, which of the bending wires 23 for R (right), L (left), U (up) or D (down) bending.

According to the first embodiment, the bending mechanism 20 is configured such that the bending wires 23 are inserted respectively through the tubes 24 each having the surface with good sliding property, and then the tubes 24 through which the bending wires 23 are respectively inserted are inserted through the wire receivers 22. With such a configuration, each of the bending wires 23 does not directly contact the wire receivers 22, which prevents friction and catching from occurring between the unevenness on the surface of each of the bending wires 23 and the inner circumferential surface and the corner portion 22r of each of the wire receivers 22.

The friction coefficient between the bending wires 23 and the tubes 24 is smaller than the friction coefficient between the bending wires 23 and the wire receivers 22. Such a configuration can reduce a loss of the pulling force of the bending wires 23 caused by the wire receivers 22. In addition, the configuration can reduce the abrasion, the scraping, and the like that occur in the corner portion 22r of each of the wire receivers 22.

Furthermore, the sliding resistance of the bending wires 23 can be reduced without using a power lubricant. Since no powder lubricant is used, the endoscope 1 can be manufactured in a controlled area such as a clean room. This improves the degree of freedom of manufacturing, reduces the manufacturing costs, and simplifies the construction of the assembly line.

In addition, since no powder lubricant is used, the configuration of the present embodiment can be applied also to the case where the endoscope 1 is a single-use endoscope which is not water-tightly sealed. Therefore, a design of water-tight sealing can be omitted for a part of the endoscope 1, and the endoscopes can be designed more inexpensively.

Note that also a configuration can be considered in which a loss of the pulling force due to friction is reduced by applying resin coating to the surfaces of the bending wires 23 each formed of the twisted wire. However, even if the resin coating is applied, it is difficult to completely smooth the surface shape of the bending wires 23. In contrast, according to the present embodiment, the tubes 24 each having a completely-smoothed surface shape can be used. With this, the sliding resistance that occurs between the tubes 24 and the wire receivers 22 in the present embodiment becomes smaller than the sliding resistance that occurs between the bending wires 23 to which the resin coating is applied and the wire receivers 22.

Thus, the present embodiment can provide the inexpensive bending mechanism 20 that enables the bending operation to be performed easily with a small amount of operation force, and the endoscope 1 including the bending mechanism 20.

According to the first embodiment, even when the bending portion 2b is maximally bent, the tubes 24 do not fall off from the coil pipes 25, and the distal ends of the coil pipes 25 are prevented from interfering with the proximal ends of the tubes 24. In addition, the proximal ends of the tubes 24 can be prevented from being caught by inner circumferential wall surfaces of the coil pipes 25.

Furthermore, each of the tubes 24 is arranged only on the distal end side in the tubular portion 2c, and not arranged over the entire length of the tubular portion 2c. Such a configuration can reduce the moving amount of the proximal end, which is the free end, of each of the tubes 24 that advances or retreats in the direction of the center axis O, when the shape of the tubular portion 2c is changed due to the sagging thereof.

In general, the entire length of the tubular portion 2c is almost equal to the entire length of the insertion portion 2, and the entire length of the bending portion 2b is remarkably shorter than the entire length of the tubular portion 2c. In the configuration of the present embodiment, the entire length of each of the tubes 24 is remarkably shorter than the entire length of the insertion portion 2. Therefore, the present embodiment can reduce the sliding resistance between the bending wires 23 and the tubes 24 and reduce also the sliding resistance between the tubes 24 and the coil pipes 25, compared to a configuration in which the tubes 24 are provided up to the operation portion 3 side (see a sixth embodiment to be described later).

### [Second Embodiment]

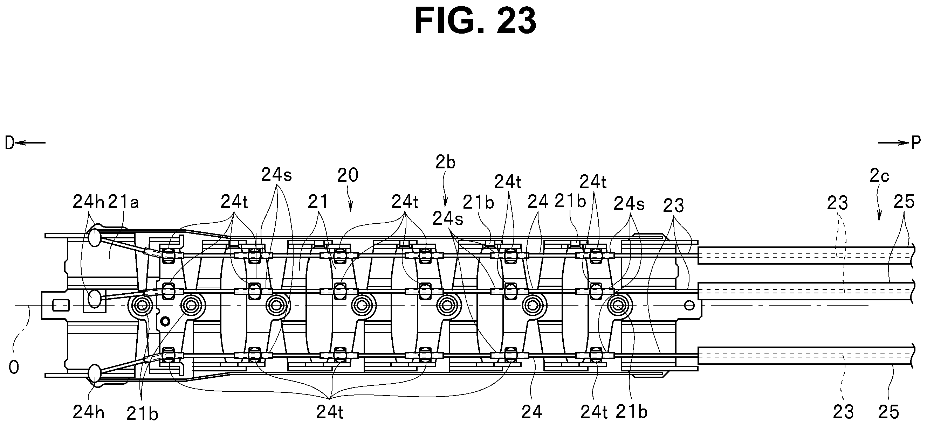

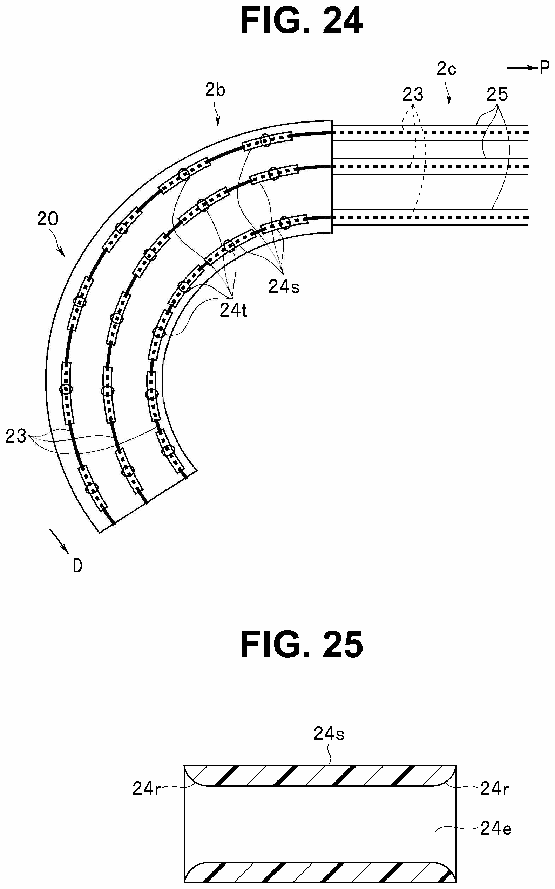

FIG. 23 to FIG. 25 show a second embodiment of the present disclosure. FIG. 23 is a cross-sectional view showing a configuration of tubes 24 through which bending wires 23 are respectively inserted in the bending portion 2b in a straight state in the second embodiment. FIG. 24 is a cross-sectional view showing a state of the tubes 24 through which the bending wires 23 are respectively inserted in the bending portion 2b in a maximum bending state in the second embodiment.

In the second embodiment, the same components as those in the first embodiment and the related technology are attached with the same reference signs and descriptions thereof will be omitted as appropriate. In the second embodiment, description will be made mainly on points different from the first embodiment and the related technology.

The distal end (end on a second side) of each of the bending wires 23 is fixed to the distal end side of the bending portion 2b, and fixed, for example, to the bending piece 21a arranged at the distal-most position at a fixing portion 24h. The fixing portion 24h is different from the fixing portion 24f in the first embodiment, and it fixes only the distal end of each of the bending wires 23 and does not fix the tube 24.

Similarly to the first embodiment, a plurality of wire receivers 22 (second guides) are provided respectively to the plurality of bending pieces 21a.

The tubes 24 (first guides) in the present embodiment each include a plurality of partial tubes 24s that are provided intermittently along the center axis O. The number of the partial tubes 24s related to a certain bending direction is the same as the number of the wire receivers 22 related to the same bending direction as the certain bending direction. Hereinafter, the number of the partial tubes 24s is represented by a natural number n.

The n wire receivers 22 fix n partial tubes 24s respectively at n fixing portions 24t. In other words, also the number of the fixing portion 24t related to the certain bending direction is the same as the number of the partial tubes 24s and the number of the wire receivers 22, these numbers being related to the same bending direction as the certain bending direction.

Note that the wire receivers 22 may be fixed respectively to the plurality of bending pieces 21 aligned along the center axis O (axis). In addition, the wire receivers 22 may be fixed to the plurality of bending pieces 21 aligned along the center axis O (axis) at appropriate intervals. As specific examples, the wire receivers 22 may be fixed respectively only to the even-number bending pieces 21 counted from the distal end side, may be fixed to the bending pieces 21 at a rate of one in three, or fixed in another arranging manner.

The total sum of the lengths, which are in the direction along the center axis O, of all the partial tubes 24s arranged on one bending wire 23 is shorter than the length of the bending wire 23 which is on the inner circumferential side in the bending portion 2b when the bending portion 2b is in the maximum bending state, as shown in FIG. 24.

Furthermore, for all of the partial tubes 24s arranged on one bending wire 23, the length and the arrangement interval of each of the partial tubes 24s are set such that the partial tubes 24s adjacent to each other do not interfere with each other (the positions thereof in the direction along the center axis O do not overlap each other) when the bending portion 2b is in the maximum bending state.

In addition, a sufficient distance is provided between the proximal end of the partial tube 24s arranged on the proximal-most side and the distal end of each of the coil pipes 25. Therefore, regardless of the bending state, the partial tubes 24s and the coil pipes 25 do not interfere with each other.

According to the second embodiment, for one bending wire 23, the total sum of the lengths of the n partial tubes 24s provided intermittently is shorter than the length of the single tube 24 provided as only one tube. Therefore, the total length of the contacting parts of the bending wire 23, which are in contact with the partial tubes 24s, is shortened than the length of the contacting part of the bending wire 23, which is in contact with the single tube 24. The length of the contacting parts is shortened, thereby enabling the sliding resistance between the bending wire 23 and the n partial tubes 24s to be reduced. As a result, the amount of operation force can be reduced, which can improve the operability.

Furthermore, the n partial tubes 24s are fixed respectively to the n wire receivers 22 at the n fixing portions 24t. In such a configuration, the partial tubes 24s do not move relatively to the wire receivers 22 to which the partial tubes 24s are fixed, that is, no sliding resistance occurs between the partial tubes 24s and the wire receivers 22.

FIG. 25 is a cross-sectional view parallel to the center axis O, which shows a modified example of a partial tube 24s constituting an intermittent tube 24 in the second embodiment.

The partial tube 24s includes, inside thereof, an insertion hole 24e for inserting a bending wire 23. In the second embodiment, the plurality of partial tubes 24s are arranged on one bending wire 23. If edges exist respectively at the entrance and the exit of each of the insertion holes 24e, the edges whose number is twice (2n) the number (n) of the partial tubes 24s arranged on the one bending wire 23, may be likely to contact the bending wire 23. The total number of these edges is n times the number (two) of the edges in the case where only the single tube 24 is arranged over the one bending wire 23.

In view of the above, R-shaped portions 24r are provided respectively at the entrances and the exits of the insertion holes 24e of all the partial tubes 24s, to round the edges, thereby reducing the sliding resistance.

According to the modified example of the second embodiment, the sliding resistance between the bending wire 23 and the n partial tubes 24s is further reduced, to further decrease the amount of operation force, which enables the operability to be improved.

Note that the configuration in which the R-shaped portions 24r are provided respectively at the entrance and the exit of each of the insertion holes 24e may be applied to a tube 24 according to other embodiments.

### [Third Embodiment]

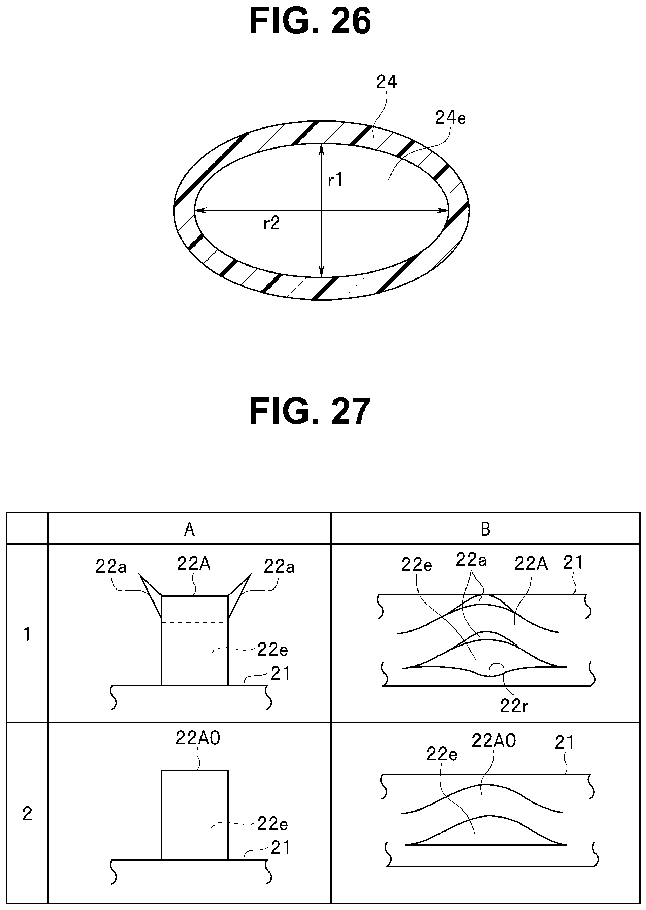

FIG. 26 is a cross-sectional view vertical to the center axis, which shows a configuration of tubes 24 through which bending wires 23 are respectively inserted in a third embodiment of the present disclosure.

In the third embodiment, the same components as those in the first and second embodiments and the related technology are attached with the same reference signs and descriptions thereof will be omitted as appropriate. In the third embodiment, description will be made mainly on points different from the first and second embodiments and the related technology.

The tube 24 in the present embodiment is formed in a flat shape so as to include a minor axis r1 in a radial direction in the cross section vertical to the center axis O. The tube 24 further includes a major axis r2 in a radial direction different from the minor axis t1. In FIG. 26, the minor axis r1 and the major axis r2 are shown respectively as a short diameter and a long diameter inside an insertion hole 24e whose cross section forms an elliptic shape.

In the example shown in the drawing, the cross section of the tube 24, which is vertical to the center axis O, becomes the elliptic shape. However, it is not limited to the example, and the cross section of the tube 24 may be any shape as long as the shape of the cross section has different lengths in the radial directions.

As the bending angle becomes large, deformation of the tube 24 becomes great according to the magnitude of the bending angle. Then, a gap between the inner wall of the tube 24 and the bending wire 23 becomes narrow, and the inner wall of the tube 24 and the bending wire 23 contact each other, which can be considered to result in an increase in the sliding resistance.

To address this, according to the third embodiment, the tube 24 having a non-circular cross section including the minor axis r1 and the major axis r2 is used, which allows the bending wire 23 to escape toward the major axis r2 side in the tube 24 at the time of bending. Such a configuration reduces the sliding resistance when the bending wire 23 is pulled, to thereby be capable of suppressing the deterioration in the operability.

### [Fourth Embodiment]

FIG. 27 is a chart showing a configuration example in which wire receivers 22A and 22A0 are each provided integrally with a bending piece 21 in a fourth embodiment of the present disclosure. In FIG. 27, the first column shows an example of the wire receiver 22A according to the present embodiment, and the second column shows an example of the general wire receiver 22A0 in comparison with the present embodiment. In addition, in FIG. 27, the column A is a partial side view of the wire receivers 22A and 22A0 viewed in a direction vertical to the center axis O, and the column B is a partial perspective view of the wire receivers 22A and 22A0 viewed in a front direction along the center axis O.

In the fourth embodiment, the same components as those in the first to third embodiments and the related technology are attached with the same reference signs and descriptions thereof will be omitted as appropriate. In the fourth embodiment, description will be made mainly on points different from the first to third embodiments and the related technology.

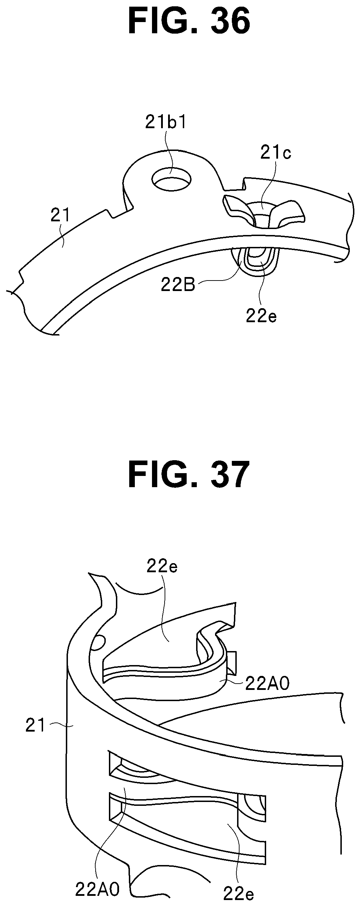

Each of the wire receivers is provided separately from the bending piece 21 (see FIG. 36 to be described later) in some cases, and provided integrally with the bending piece 21 (see FIG. 37 to be described later) in other cases. The present embodiment falls under the case where each of the wire receivers is provided integrally.

In this case, the bending pieces 21 constituting the bending portion 2b, and the wire receivers 22A and 22A0 are formed of the same material.

The wire receiver 22A0 shown in the second column is formed, for example, by making two cuts in a part of a cylindrical body constituting the bending piece 21 in the circumferential direction, and deforming the parts where the cuts are made radially inward by press working, for example.

Between the wire receiver 22A0 and the cylindrical body constituting the bending piece 21, an insertion hole 22e is formed for inserting the bending wire 23 held by the tube 24.

The wire receiver 22A shown in the first column is formed basically in the same manner as the wire receiver 22A0. In other words, the wire receiver 22A is formed, for example, by making two cuts in a part of the cylindrical body constituting the bending piece 21 in the circumferential direction, and deforming the parts where the cuts are made radially inward by press working, for example.

Meanwhile, the wire receiver 22A is different from the wire receiver 22A0 in that tongue pieces 22a are further formed respectively at the entrance and the exit of the insertion hole 22e when the press working is performed. On the other hand, at the positions opposed to the tongue pieces 22a at the entrance and the exit of the insertion hole 22e, corner portions 22r rounded in an R-shape are formed.

According to the fourth embodiment, when the wire receiver 22A is formed by the press working, the tongue pieces 22a are further formed. In such a configuration, even if the tube 24 advances or retreat relatively to the wire receiver 22A in the direction of the center axis O at the time of bending, the sliding resistance to occur can be reduced. As a result, the amount of operation force required for operating the bending operation knob 3b is decreased, to thereby enable the operability to be improved.

### [Fifth Embodiment]

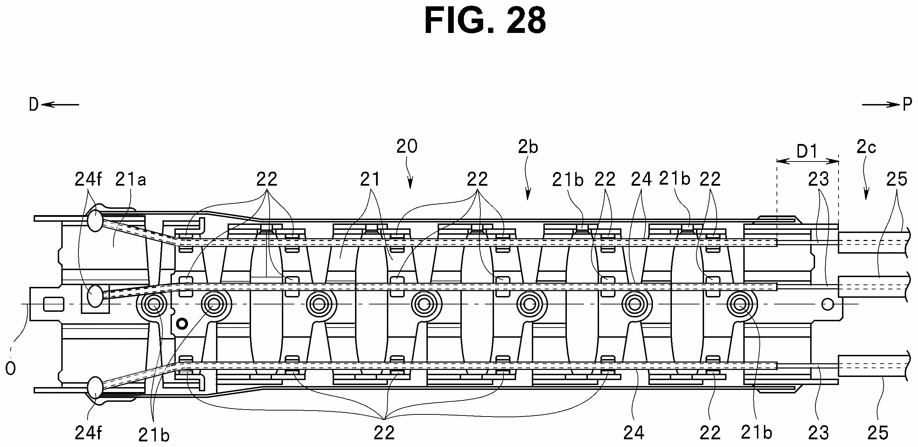

FIG. 28 shows a configuration of tubes 24 through which bending wires 23 are respectively inserted in a fifth embodiment of the present disclosure. In the fifth embodiment, the tubes 24 are arranged only in the bending portion 2b.

In the fifth embodiment, the same components as those in the first to fourth embodiments and the related technology are attached with the same reference signs and descriptions thereof will be omitted as appropriate. In the fifth embodiment, description will be made mainly on points different from the first to fourth embodiments and the related technology.

The distal end of each of the tubes 24 is, together with the distal end of each of the bending wires 23, fixed to the bending piece 21a at the fixing portion 24f. The tubes 24 are each inserted through the plurality of wire receivers 22, and further inserted through the proximal-most wire receiver 22, and thereafter end in the bending portion 2b.

When each of the bending wires 23 extended from the tube 24 in the bending portion 2b reaches the tubular portion 2c, it is inserted into the coil pipe 25.

When each of the bending wires 23 is pulled, the distal end, which is the fixed end, of each of the tubes 24 does not move, and the proximal end, which is the free end, of each of the tubes 24 moves in the axial direction. For example, the proximal end of the tube 24, which is located on the inner circumferential side when the bending portion 2b is bent, moves toward the proximal end side (tubular portion 2c side). Even at the time of such movement, in order to prevent the proximal end of each of the tubes 24 from contacting the distal end of each of the coil pipes 25, a predetermined distance D1 is provided between the proximal end (end on the first side (proximal end direction P side)) of each of the tubes 24 (first guides) and the distal end (end on the second side (distal end direction D side)) of each of the coil pipes 25 (third guides).

In addition, the proximal end of the tube 24 which is located on the outer circumferential side when the bending portion 2b is bent moves toward the distal end side (distal end portion 2a side). The length of the tube 24 is adjusted such that the proximal end of the tube 24 does not fall off from the proximal-most wire receiver 22 even at the time of the movement.

In other words, considering both cases where the tube 24 is located on the bending inner circumferential side and located on the bending outer circumferential side, the length of the tube 24 is adjusted. Specifically, the length of the tube 24 is adjusted such that the proximal end of the tube 24 is located at substantially the middle position between the proximal-most wire receiver 22 and the distal end of the coil pipe 25, when the entire insertion portion 2 is in a straight state. In this case, the length of the tube 24 is a little shorter than the entire length of the bending portion 2b.

According to the fifth embodiment, similarly to the above-described respective embodiments, the sliding resistance can be reduced, the amount of operation force can be reduced, and the operability can be improved. In addition, the proximal end of each of the tubes 24 can be prevented from interfering with the wire receivers 22 and each of the coil pipes 25.

### [Sixth Embodiment]

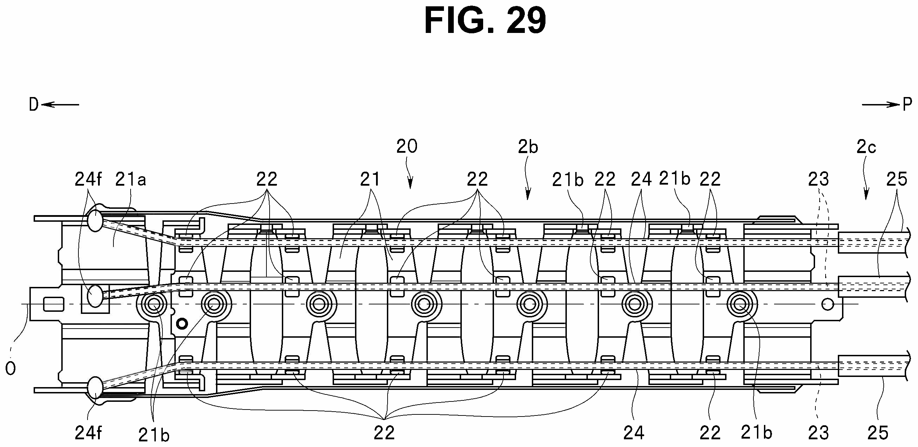

FIG. 29 shows a configuration of tubes 24 through which bending wires 23 are respectively inserted in a sixth embodiment of the present disclosure. In the sixth embodiment, the tubes 24 are arranged both in the bending portion 2b and the tubular portion 2c.

In the sixth embodiment, the same components as those in the first to fifth embodiments and the related technology are attached with the same reference signs and descriptions thereof will be omitted as appropriate. In the sixth embodiment, description will be made mainly on points different from the first to fifth embodiments and the related technology.

The distal end of each of the tubes 24 is, together with the distal end of each of the bending wires 23, fixed to the bending piece 21a at the fixing portion 24f. Each of the tubes 24 is inserted into the inside of the bending portion 2b and further inserted into the inside of the coil pipe 25 in the tubular portion 2c. Although illustration is omitted, on the proximal end side of the tubular portion 2c, the proximal end of each of the tubes 24 is extended from the coil pipe 25. The proximal end side of each of the tubes 24 can be moved into the operation portion 3.

The sixth embodiment provides substantially the same effects as those of the fifth embodiment.

In addition, each of the coil pipes 25 has a spiral-shaped unevenness on the inner surface thereof. Therefore, the bending wires 23 each formed of a twisted wire, is caught by the unevenness on the inner surface of each of the coil pipes 25 when each of the bending wires 23 is pulled, which may possibly increase the sliding resistance.

To address this, according to the sixth embodiment, the tubes 24 are each arranged also inside the coil pipe 25, which can suppress the increase of the sliding resistance in the coil pipe 25 when the bending wire 23 is pulled. Thus, the sliding resistance in the entire insertion portion 2 can be reduced, thereby enabling the amount of operation force of the bending operation knob 3b to be further reduced.

### [Seventh Embodiment]

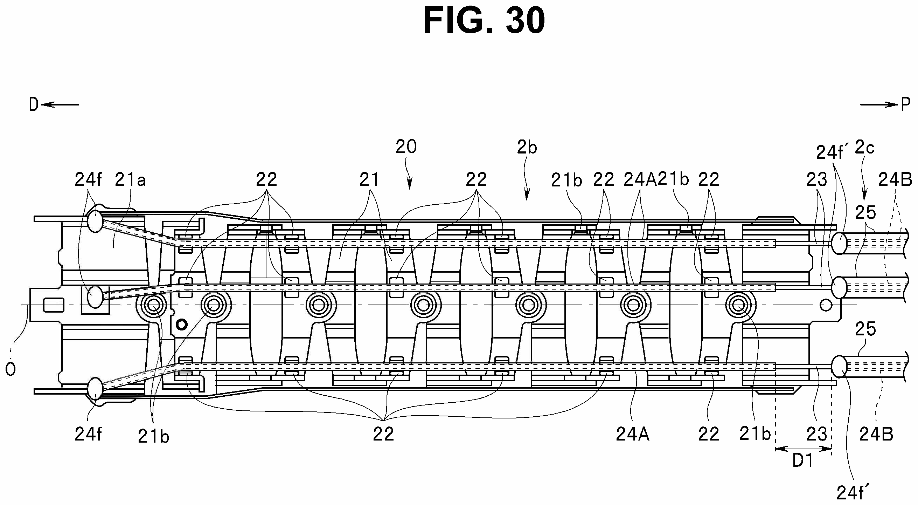

FIG. 30 shows a configuration of tubes 24 through which bending wires 23 are respectively inserted in a seventh embodiment of the present disclosure. In the seventh embodiment, as a tube 24, a first tube 24A (first guide) and a second tube 24B (fourth guide) are provided, and the first tube 24A is inserted into the bending portion 2b to be arranged therein and the second tube 24B is inserted into the tubular portion 2c to be arranged therein.

In the seventh embodiment, the same components as those in the first to sixth embodiments and the related technology are attached with the same reference signs and descriptions thereof will be omitted as appropriate. In the seventh embodiment, description will be made mainly on points different from the first to sixth embodiments and the related technology.

The first tube 24A is configured similarly to the tube 24 in the fifth embodiment shown in FIG. 28. In other words, the distal end of the first tube 24A is, together with the distal end of the bending wire 23, fixed to the bending piece 21a at the fixing portion 24f. In addition, the length of the first tube 24A is adjusted such that the proximal end of the first tube 24A is located at substantially the middle position between the proximal-most wire receiver 22 and the distal end of the coil pipe 25, when the entire insertion portion 2 is in the straight state.

The end (distal end) on the distal end direction D side (second side) of the second tube 24B (fourth guide) is, together with the distal end of the coil pipe 25, fixed at a second fixing portion 24f´, in a boundary portion between the bending portion 2b (first tube) and the tubular portion 2c (second tube). Note that the second fixing portion 24f´ does not fix the bending wire 23.

The second tube 24B is inserted through the coil pipe 25, and can be extended from the coil pipe 25 on the proximal end side of the tubular portion 2c, similarly to the tube 24 in the sixth embodiment described with reference to FIG. 29. Accordingly, the proximal end of the second tube 24B can be moved into the operation portion 3.

Similarly to the example shown in FIG. 28, a predetermined distance D1 is provided between the proximal end of the first tube 24A and the second fixing portion 24f´ to which the distal end of the coil pipe 25 is fixed.

The seventh embodiment provides substantially the same effects as those of the sixth embodiment.

Furthermore, according to the seventh embodiment, the first tube 24A is arranged in the bending portion 2b and the second tube 24B is arranged in the tubular portion 2c. With such a configuration, an influence of advancing and retreating of the first tube 24A according to the bending of the bending portion 2b can be absorbed in the bending portion 2b and an influence of advancing and retreating of the second tube 24B according to the sagging of the tubular portion 2c can be absorbed in the tubular portion 2c. Therefore, compared to the tube 24 in the sixth embodiment, the advancing and retreating lengths of the first tube 24A and the second tube 24B can be reduced.

Accordingly, the respective sliding resistances generated by the first tube 24A and the second tube 24B each contacting the internal components of the insertion portion 2 can be made smaller than the sliding resistance generated by the one tube 24 contacting the internal components in the sixth embodiment.

### [Eighth Embodiment]

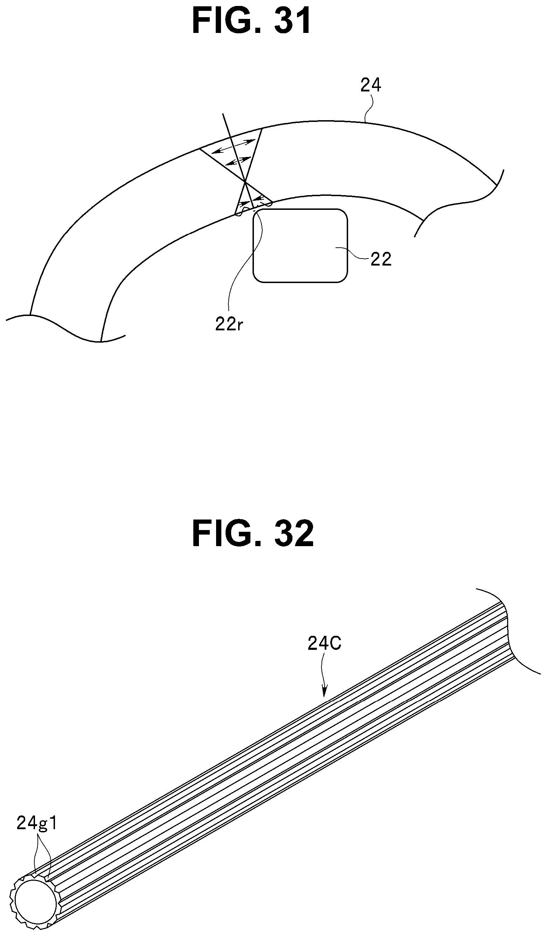

FIG. 31 shows a state of a tube 24, when the bending portion 2b is bent, in an eighth embodiment of the present disclosure.

In the eighth embodiment, the same components as those in the first to seventh embodiments and the related technology are attached with the same reference signs and descriptions thereof will be omitted as appropriate. In the eighth embodiment, description will be made mainly on points different from the first to seventh embodiments and the related technology.

When the bending portion 2b is bent, the tube 24 inserted through the wire receivers 22 bends in accordance with the bending shape of the bending portion 2b. At this time, the tube 24 extends on the bending outer circumferential side and contracts on the bending inner circumferential side.

As described above, the tube 24 is made of a material which is less prone to wrinkle even if the tube 24 is contracted and has a thickness set such that a wrinkle is unlikely to occur. However, when the bending radius is small, the contraction amount cannot be absorbed only by an elastic deformation of the material, and a wrinkle is likely to occur on the inner circumferential side of the bent tube 24. When a wrinkle occurs in the tube 24, the tube 24 may be caught by the corner portion 22r of the wire receiver 22.

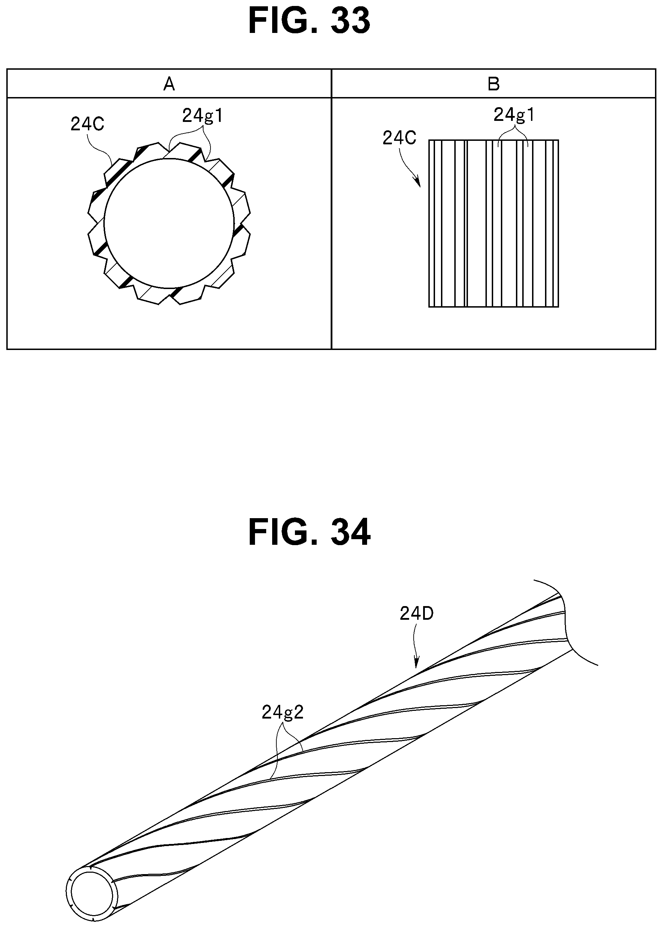

FIG. 32 is a perspective view showing a first configuration example of a tube 24C in the eighth embodiment. FIG. 33 is a chart showing a cross section and a partially enlarged lateral surface of the tube 24C in the first configuration example in the eighth embodiment. FIG. 33 shows the cross section of the tube 24C in the column A, and shows the partially enlarged lateral surface of the tube 24C in the column B.

The tube 24C includes, on the outer surface thereof, one or more grooves to prevent a wrinkle when the tube 24C is bent. In the examples shown in FIG. 32 and FIG. 33, one or more grooves 24g1, i.e., a plurality of grooves 24g1 in the illustrated examples are provided on the outer surface of the tube 24C. The grooves 24g1 are parallel to the direction of the center axis of the tube 24C (in other words, the grooves form an angle of 0 degrees with respect to the axial direction).

When the tube 24C is arranged in the bending portion 2b, the direction of the center axis of the tube 24C is parallel to the direction of the center axis O. Note that the cross-sectional shape, the depth, the number, etc., of the grooves 24g1 are not limited to the configurations shown in FIG. 32 and FIG. 33.

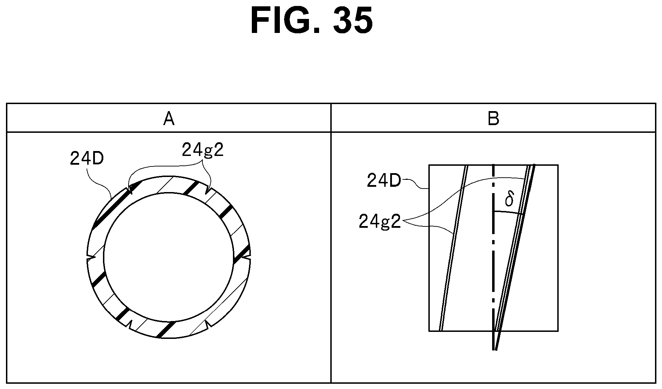

FIG. 34 is a perspective view showing a second configuration example of a tube 24D in the eighth embodiment. FIG. 35 is a chart showing a cross section and a partially enlarged lateral surface of the tube 24D in the second configuration example in the eighth embodiment. FIG. 35 shows the cross section of the tube 24D in the column A, and shows the partially enlarged lateral surface of the tube 24D in the column B.

The tube 24D includes, on the outer surface thereof, one or more grooves to prevent a wrinkle when the tube 24D is bent. In the examples shown in FIG. 34 and FIG. 35, one or more grooves 24g2, i.e., a plurality of spiral-shaped grooves 24g2 in the illustrated examples are provided on the outer surface of the tube 24D. The grooves 24g2 form an angle δ with respect to the direction of the center axis of the tube 24D. δ is an angle within a range of 0 degrees < δ ≤ 30 degrees, for example.

Here, when the tube 24D is arranged in the bending portion 2b, the direction of the center axis of the tube 24D is parallel to the direction of the center axis O. Note that the cross-sectional shape, the depth, the number, etc., of the grooves 24g2 are not limited to the configurations shown in FIG. 34 and FIG. 35.

If both of the configuration examples of the tube 24C and the tube 24D are combined, the grooves formed on the outer surface (outer circumferential surface) of the tube 24 may form an angle of 0 degrees or more and 30 degrees or less (or approximately 30 degrees or less) with respect to the direction of the axis (center axis O).

According to the eighth embodiment, the grooves 24g1 and 24g2 as shown in FIGS. 32 to 35 are provided on the outer surface of the tube 24, which can achieve a structure in which a wrinkle hardly occurs on the outer surface of the tube 24.

Furthermore, the angles of the grooves 24g1 and 24g2 with respect to the direction of the axis (center axis O) are set to 0 degrees or more and 30 degrees or less (or approximately 30 degrees or less), which provides the tube 24 whose slidability with the wire receiver 22 is not impaired.

### [Ninth Embodiment]

FIG. 36 is a partial perspective view showing a configuration of a wire receiver 22B in a bending piece 21 in a ninth embodiment of the present disclosure.

In the ninth embodiment, the same components as those in the first to eighth embodiments and the related technology are attached with the same reference signs and descriptions thereof will be omitted as appropriate. In the ninth embodiment, description will be made mainly on points different from the first to eighth embodiments and the related technology.

The bending pieces 21 each include a hinge attaching hole 21b1 and a wire receiver attaching hole 21c. Attaching a hinge to the hinge attaching hole 21b1 enables two adjacent bending pieces 21 to be connected so as to be deflectable with respect to each other.

A wire receiver 22B, which is configured separately from the cylindrical body constituting the bending piece 21, is attached to the wire receiver attaching hole 21c. The inside of the wire receiver 22B serves as an insertion hole 22e for inserting the tube 24 that houses the bending wire 23. Thus, such a configuration may be employed in which the wire receiver 22B manufactured as a separate part is attached to the bending piece 21.

Note that the bending piece 21 constituting the bending portion 2b and the wire receiver 22B may be formed of the same material or may be formed of different materials.

According to the ninth embodiment, using the wire receiver 22B, which is the part formed separately from the main body of the bending piece 21, enables the wire receiver 22B whose quality is stabilized to be manufactured inexpensively. Therefore, the bending mechanism 20 including the bending piece 21 has a configuration suitable for a single-use endoscope for which reduced costs are required.

FIG. 37 is a partial perspective view showing a configuration of a wire receiver 22A0 in a bending piece 21 in a modified example of the ninth embodiment.

As described above with reference to the second column in FIG. 27, the wire receiver 22A0 may be configured integrally with the cylindrical body constituting the bending piece 21.

In other words, as shown in FIG. 37, the wire receiver 22A0 is formed, for example, by making two cuts in a part of the cylindrical body constituting the bending piece 21 in the circumferential direction, and deforming the parts where the cuts are made radially inward by press working, for example. In this case, the wire receiver 22A0 is formed of the same material as that of the bending piece 21.

The inside of the wire receiver 22A0 serves as the insertion hole 22e for inserting the tube 24 that houses the bending wire 23.

According to the modified example of the ninth embodiment, the bending piece 21 and the wire receiver 22A0 can be integrally formed by the press working, or the like, which enables the bending piece 21 to be produced inexpensively. Therefore, the bending mechanism 20 including the bending piece 21 has a configuration suitable for a single-use endoscope for which reduced costs are required.

### [Notes]

According to the above-described descriptions related to the embodiments of the present disclosure, the following configurations can be obtained.

### [Note A1]

An endoscope including:
a flexible tube,
the flexible tube being a tube that is along a center axis extending from one end to another end,
the flexible tube including:
   a plurality of protruded surfaces provided on an outer circumferential surface of the flexible tube; and
   recessed surfaces provided on the outer circumferential surface, the recessed surfaces being defined by the plurality of protruded surfaces, wherein
   the plurality of protruded surfaces are each surrounded by the recessed surfaces, so as to be isolated from one another,
   when the recessed surfaces each include a first part along an axial direction parallel to the center axis, the first parts are formed intermittently in the axial direction, and
   when the recessed surfaces each include a second part along a circumferential direction around the center axis, the second parts are formed intermittently in the circumferential direction.

### [Note A2]

The endoscope according to Note A1, wherein
each of the plurality of protruded surfaces has a same shape.

### [Note A3]

The endoscope according to Note A2, wherein
each of the plurality of protruded surfaces forms a polygon when the outer circumferential surface is planarly developed.

### [Note A4]

The endoscope according to Note A2, wherein
each of the plurality of protruded surfaces forms a rounded polygon when the outer circumferential surface is planarly developed.

### [Note A5]

The endoscope according to Note A3, wherein
the recessed surfaces each include a part inclined with respect to the axial direction, and
the part inclined forms an angle of 45 degrees or less with respect to the axial direction.

### [Note A6]

The endoscope according to Note A3, wherein
each of the plurality of protruded surfaces forms a quadrangle when the outer circumferential surface is planarly developed.

### [Note A7]

The endoscope according to Note A6, wherein
each of the plurality of protruded surfaces forms a rhombus when the outer circumferential surface is planarly developed.

### [Note A8]

The endoscope according to Note A6, wherein
each of the plurality of protruded surfaces forms a rectangle when the outer circumferential surface is planarly developed.

### [Note A9]

The endoscope according to Note A2, wherein
each of the plurality of protruded surfaces forms a T-shape when the outer circumferential surface is planarly developed.

### [Note A10]

The endoscope according to Note A2, wherein
each of the plurality of protruded surfaces forms an L-shape when the outer circumferential surface is planarly developed.

### [Note A11]

The endoscope according to Note A3, wherein
each of the plurality of protruded surfaces forms a triangle when the outer circumferential surface is planarly developed.

### [Note A12]

The endoscope according to Note A2, wherein
the flexible tube includes:
a tube main body formed by providing the plurality of protruded surfaces and the recessed surfaces, the tube main body being made of a material having a first Young's modulus; and
filling structure parts with which the recessed surfaces on the tube main body are respectively filled, the filling structure parts being made of a filling material having a second Young's modulus lower than the first Young's modulus.

### [Note A13]

The endoscope according to Note A12, wherein
the filling structure parts have different filling rates of the filling material along the axial direction.

### [Note A14]

The endoscope according to Note A2, wherein
distances from the recessed surfaces to the plurality of protruded surfaces differ in a radial direction with the center axis as a center.

### [Note A15]

The endoscope according to Note A1, further including,
an insertion portion configured to be inserted into a subject, wherein
the flexible tube is provided in the insertion portion.

### [Note A16]

The endoscope according to Note A1, wherein
the plurality of protruded surfaces are arranged periodically along the outer circumferential surface.

### [Note A17]

The endoscope according to Note A1, wherein
the plurality of protruded surfaces are arranged aperiodically along the outer circumferential surface.

### [Note A18]

The endoscope according to Note A1, wherein
the recessed surfaces each include at least one of the first part or the second part.

### [Note A19]

The endoscope according to Note A1, wherein
each of the plurality of protruded surfaces has a first shape in a first range along the axial direction, and
each of the plurality of protruded surfaces has a second shape different from the first shape in a second range along the axial direction, the second range being different from the first range.

### [Note A20]

An endoscope including,
a flexible tube,
the flexible tube being a tube that is along a center axis extending from one end to another end, the flexible tube including:
   a plurality of protruded surfaces provided on an outer circumferential surface of the flexible tube; and
   recessed surfaces provided on the outer circumferential surface, the recessed surfaces being defined by the plurality of protruded surfaces, wherein
the plurality of protruded surfaces are each surrounded by the recessed surfaces, so as to be isolated from one another, and
the recessed surfaces are all inclined with respect to an axial direction parallel to the center axis, and inclined with respect to a circumferential direction around the center axis.

### [Note B1]

An endoscope including:
a flexible tube,
the flexible tube being a tube that is along a center axis extending from one end to another end,
the flexible tube including:
   a plurality of protruded surfaces provided on an outer circumferential surface of the flexible tube; and
   recessed surfaces provided on the outer circumferential surface, the recessed surfaces being defined by the plurality of protruded surfaces, wherein
the plurality of protruded surfaces are each surrounded by the recessed surfaces, so as to be isolated from one another, and
the recessed surfaces are all inclined with respect to an axial direction parallel to the center axis, and inclined with respect to a circumferential direction around the center axis.

### [Note B2]

The endoscope according to Note B1, wherein
each of the plurality of protruded surfaces forms a quadrangle when the outer circumferential surface is planarly developed.

### [Note B3]

The endoscope according to Note B1, wherein
each of the plurality of protruded surfaces forms a rhombus when the outer circumferential surface is planarly developed.

### [Note B4]

The endoscope according to Note B1, wherein
each of the plurality of protruded surfaces forms a rectangle when the outer circumferential surface is planarly developed.

### [Note B5]

A flexible tube that is a tube along a center axis extending from one end to another end, the flexible tube including:
a plurality of protruded surfaces provided on an outer circumferential surface of the flexible tube; and
recessed surfaces provided on the outer circumferential surface, the recessed surfaces being defined by the plurality of protruded surfaces, wherein
the plurality of protruded surfaces are each surrounded by the recessed surfaces, so as to be isolated from one another,
when the recessed surfaces each include a first part along an axial direction parallel to the center axis, the first parts are formed intermittently in the axial direction, and
when the recessed surfaces each include a second part along a circumferential direction around the center axis, the second parts are formed intermittently in the circumferential direction.

Note that the present disclosure is not limited as-is to the above described embodiments. It is possible to embody the present disclosure by modifying the constituent elements in a range without departing from the gist of the disclosure at the practical stage. In addition, various aspects of the disclosure can be achieved by appropriately combining the plurality of constituent elements disclosed in the above-described embodiments. Some of the constituent elements may be deleted from all the constituent elements disclosed in the embodiments, for example. Furthermore, constituent elements over different embodiments may be combined as appropriate. It goes without saying that various modifications and applications can be implemented within a range without departing from the gist of the disclosure.

The present application is filed claiming the benefit of priority to U.S. provisional Application No. 63/599292 filed on November 15, 2023, the contents of which are incorporated by this reference in the description, claims, and drawings of this application.

## Claims

1. A bending mechanism comprising:
a first tube formed along an axis extending from a first side to a second side, the first tube being configured to be bent by a wire being pulled;
a first guide made of a resin and formed in a cylindrical shape, the first guide being provided in the first tube along the axis and configured to hold the wire; and
a second guide fixed to the first tube, and configured to hold the first guide.

2. The bending mechanism according to claim 1, wherein
the first tube includes:
a plurality of cylinders aligned along the axis; and
a connecting mechanism that connects two adjacent cylinders of the plurality of cylinders such that one of the two adjacent cylinders is deflectable with respect to the other, wherein
the connecting mechanism is formed separately from the first guide.

3. The bending mechanism according to claim 1, wherein
the resin is HDPE, that is, high-density polyethylene, or PTFE, that is, polytetrafluoroethylene.

4. The bending mechanism according to claim 1, further comprising
the wire, wherein
the wire is inserted through the first guide,
an end of the wire on the second side is fixed to the second side of the first tube, and
a friction coefficient between the wire and the first guide is smaller than a friction coefficient between the wire and the second guide.

5. The bending mechanism according to claim 1, wherein
the first guide includes a groove on an outer circumferential surface of the first guide, the groove forming an angle of 30 degrees or less with respect to a direction of the axis.

6. The bending mechanism according to claim 1, further comprising:
a second tube provided along the axis, on the first side relative to the first tube; and
a third guide arranged along the axis, in the second tube, wherein
the first guide is arranged, from the first tube, so as to enter the third guide up to a halfway position of the third guide, and the first guide is provided such that an entered length of the first guide into the third guide is a first predetermined length when the first tube is in a straight state.

7. The bending mechanism according to claim 6, wherein
the first predetermined length is set to such a length that the first guide does not fall off from the third guide even in a state where the first tube is maximally bent, based on a length, in a radial direction, from a center axis of the first tube to a center of the second guide, a length of the first tube in a direction of the axis, and a bending angle when the first tube is maximally bent.

8. The bending mechanism according to claim 6, wherein
in a state where the first tube is maximally bent, a second predetermined length of the first guide entered into the third guide is set to 1 mm or more and 20 mm or less.

9. The bending mechanism according to claim 6, wherein
in a state where the first tube is maximally bent, a second predetermined length of the first guide entered into the third guide is set to 10 mm or more.

10. The bending mechanism according to claim 1, wherein
the first guide includes a plurality of partial guides provided intermittently along the axis, and
the second guide includes a plurality of second guides, and the plurality of second guides fix the plurality of partial guides, respectively.

11. The bending mechanism according to claim 10, wherein
the first tube includes:
a plurality of cylinders aligned along the axis; and
a connecting mechanism that connects two adjacent cylinders of the plurality of cylinders such that one of the two adjacent cylinders is deflectable with respect to the other, and
the plurality of second guides are fixed to the plurality of cylinders, respectively.

12. The bending mechanism according to claim 1, wherein
the first guide is formed in a flat shape so as to include a minor axis in a radial direction, in a cross section vertical to the axis.

13. The bending mechanism according to claim 1, wherein
the first tube and the second guide are formed of a same material.

14. The bending mechanism according to claim 1, further comprising:
a second tube provide along the axis, on the first side relative to the first tube; and
a third guide arranged along the axis, in the second tube, wherein
an end of the first guide on the first side is arranged such that a predetermined distance is provided between the end of the first guide on the first side and an end of the third guide on the second side.

15. The bending mechanism according to claim 14, further comprising
a fourth guide inserted into the third guide, an end of the fourth guide on the second side being fixed to a boundary between the first tube and the second tube.

16. The bending mechanism according to claim 1, wherein
the first tube is formed of metal.

17. The bending mechanism according to claim 1, wherein
the first guide is formed to be more flexible than the second guide.

18. An endoscope comprising:
an insertion portion configured to be inserted into a subject;
an operation portion provided on a first side relative to the insertion portion; and
a bending mechanism, at least a part of which is provided on a second side of the insertion portion, wherein
the bending mechanism comprises:
a first tube formed along an axis extending from the first side to the second side, the first tube being configured to be bent by a wire being pulled;
a first guide made of a resin and formed in a cylindrical shape, the first guide being provided in the first tube along the axis and configured to hold the wire; and
a second guide fixed to the first tube, and configured to hold the first guide, and
the first side of the wire is connected to the operation portion.
